# EUROPEAN PATENT APPLICATION

(11) **EP 2 462 949 A2**
(43) Date of publication of application: **13.06.2012**
(21) Application number: 12158035.1
(22) Date of filing: 17.10.2008
(51) Int. Cl.: A61K 39/095

(54) **Meningococcal vaccine formulations**

(30) Priority: 19.10.2007 US 999590 P
(62) Divisional of application: 08838605.7
(71) Applicant: Novartis AG, 4056 Basel (CH)
(72) Inventor: Contorni, Mario, 53100 Siena (IT); Kazzaz, Jina, Cambridge, MA Massachusetts 02139 (US); O'Hagan, Derek, Cambridge, MA Massachusetts 02139 (US); Singh, Manmohan, Cambridge, MA Massachusetts 02139 (US); Ugozzoli, Mildred, Cambridge, MA Massachusetts 02139 (US)
(74) Representative: Carpmaels & Ransford

(57) **Abstract**

A lyophilised formulation for vaccines against *Neisseria meningitidis* comprises (i) an immunogen for raising an immune response against *N.meningitidis* serogroup B, and (ii) a conjugated capsular saccharide from one or more of *N.meningitidis* serogroups A, C, W135 and/or Y. The lyophilised antigens can be reconstituted into liquid form at the time of use ready for administration to a patient, and reconstitution can use an adjuvant comprising an oil-in-water emulsion. The formulation gives excellent result in terms of both stability and immunogenicity.

SEQUENCE LISTING

<110>   NOVARTIS AG

<120>   MENINGOCOCCAL VACCINE FORMULATIONS

<130>   P058737EP

<150>   EP-08838605.7
<151>   2008-10-17

<150>   PCT/IB2008/003283
<151>   2008-10-17

<150>   US-60/999,590
<151>   2007-10-19

<160>   7

<170>   SeqWin99, version 1.02

<210>   1
<211>   248
<212>   PRT
<213>   Neisseria meningitidis

<400>   1
Val Ala Ala Asp Ile Gly Ala Gly Leu Ala Asp Ala Leu Thr Ala Pro
1               5                   10                  15

Leu Asp His Lys Asp Lys Gly Leu Gln Ser Leu Thr Leu Asp Gln Ser
            20                  25                  30

Val Arg Lys Asn Glu Lys Leu Lys Leu Ala Ala Gln Gly Ala Glu Lys
        35                  40                  45

Thr Tyr Gly Asn Gly Asp Ser Leu Asn Thr Gly Lys Leu Lys Asn Asp
    50                  55                  60

Lys Val Ser Arg Phe Asp Phe Ile Arg Gln Ile Glu Val Asp Gly Gln
65                  70                  75                  80

Leu Ile Thr Leu Glu Ser Gly Glu Phe Gln Val Tyr Lys Gln Ser His
                85                  90                  95

Ser Ala Leu Thr Ala Phe Gln Thr Glu Gln Ile Gln Asp Ser Glu His
            100                 105                 110

Ser Gly Lys Met Val Ala Lys Arg Gln Phe Arg Ile Gly Asp Ile Ala
        115                 120                 125

Gly Glu His Thr Ser Phe Asp Lys Leu Pro Glu Gly Gly Arg Ala Thr
    130                 135                 140

Tyr Arg Gly Thr Ala Phe Gly Ser Asp Asp Ala Gly Gly Lys Leu Thr
145                 150                 155                 160

Tyr Thr Ile Asp Phe Ala Ala Lys Gln Gly Asn Gly Lys Ile Glu His
                165                 170                 175

Leu Lys Ser Pro Glu Leu Asn Val Asp Leu Ala Ala Ala Asp Ile Lys
            180                 185                 190

Pro Asp Gly Lys Arg His Ala Val Ile Ser Gly Ser Val Leu Tyr Asn
        195                 200                 205

Gln Ala Glu Lys Gly Ser Tyr Ser Leu Gly Ile Phe Gly Gly Lys Ala
    210                 215                 220

Gln Glu Val Ala Gly Ser Ala Glu Val Lys Thr Val Asn Gly Ile Arg
225                 230                 235                 240

His Ile Gly Leu Ala Ala Lys Gln
                245

<210>   2
<211>   247
<212>   PRT
<213>   Neisseria meningitidis

<400>   2
Val Ala Ala Asp Ile Gly Ala Gly Leu Ala Asp Ala Leu Thr Ala Pro
1               5                   10                  15

Leu Asp His Lys Asp Lys Ser Leu Gln Ser Leu Thr Leu Asp Gln Ser
            20                  25                  30

Val Arg Lys Asn Glu Lys Leu Lys Leu Ala Ala Gln Gly Ala Glu Lys
        35                  40                  45

Thr Tyr Gly Asn Gly Asp Ser Leu Asn Thr Gly Lys Leu Lys Asn Asp
    50                  55                  60

Lys Val Ser Arg Phe Asp Phe Ile Arg Gln Ile Glu Val Asp Gly Gln
65                  70                  75                  80

Leu Ile Thr Leu Glu Ser Gly Glu Phe Gln Ile Tyr Lys Gln Asp His
                85                  90                  95

Ser Ala Val Val Ala Leu Gln Ile Glu Lys Ile Asn Asn Pro Asp Lys
            100                 105                 110

Ile Asp Ser Leu Ile Asn Gln Arg Ser Phe Leu Val Ser Gly Leu Gly
        115                 120                 125

Gly Glu His Thr Ala Phe Asn Gln Leu Pro Asp Gly Lys Ala Glu Tyr
    130                 135                 140

His Gly Lys Ala Phe Ser Ser Asp Asp Ala Gly Gly Lys Leu Thr Tyr
145                 150                 155                 160

Thr Ile Asp Phe Ala Ala Lys Gln Gly His Gly Lys Ile Glu His Leu
                165                 170                 175

Lys Thr Pro Glu Gln Asn Val Glu Leu Ala Ala Ala Glu Leu Lys Ala
            180                 185                 190

Asp Glu Lys Ser His Ala Val Ile Leu Gly Asp Thr Arg Tyr Gly Ser
        195                 200                 205

Glu Glu Lys Gly Thr Tyr His Leu Ala Leu Phe Gly Asp Arg Ala Gln
    210                 215                 220

Glu Ile Ala Gly Ser Ala Thr Val Lys Ile Gly Glu Lys Val His Glu
225                 230                 235                 240

Ile Gly Ile Ala Gly Lys Gln
                245

<210>   3

<211>   250
<212>   PRT
<213>   Neisseria meningitidis

<400>   3
Val Ala Ala Asp Ile Gly Thr Gly Leu Ala Asp Ala Leu Thr Ala Pro
1               5                   10                  15

Leu Asp His Lys Asp Lys Gly Leu Lys Ser Leu Thr Leu Glu Asp Ser
            20                  25                  30

Ile Pro Gln Asn Gly Thr Leu Thr Leu Ser Ala Gln Gly Ala Glu Lys
        35                  40                  45

Thr Phe Lys Ala Gly Asp Lys Asp Asn Ser Leu Asn Thr Gly Lys Leu
    50                  55                  60

Lys Asn Asp Lys Ile Ser Arg Phe Asp Phe Val Gln Lys Ile Glu Val
65                  70                  75                  80

Asp Gly Gln Thr Ile Thr Leu Ala Ser Gly Glu Phe Gln Ile Tyr Lys
                85                  90                  95

Gln Asn His Ser Ala Val Val Ala Leu Gln Ile Glu Lys Ile Asn Asn
            100                 105                 110

Pro Asp Lys Thr Asp Ser Leu Ile Asn Gln Arg Ser Phe Leu Val Ser
        115                 120                 125

Gly Leu Gly Gly Glu His Thr Ala Phe Asn Gln Leu Pro Gly Gly Lys
    130                 135                 140

Ala Glu Tyr His Gly Lys Ala Phe Ser Ser Asp Asp Pro Asn Gly Arg
145                 150                 155                 160

Leu His Tyr Ser Ile Asp Phe Thr Lys Lys Gln Gly Tyr Gly Arg Ile
                165                 170                 175

Glu His Leu Lys Thr Leu Glu Gln Asn Val Glu Leu Ala Ala Ala Glu
            180                 185                 190

Leu Lys Ala Asp Glu Lys Ser His Ala Val Ile Leu Gly Asp Thr Arg
        195                 200                 205

Tyr Gly Ser Glu Glu Lys Gly Thr Tyr His Leu Ala Leu Phe Gly Asp
    210                 215                 220

Arg Ala Gln Glu Ile Ala Gly Ser Ala Thr Val Lys Ile Gly Glu Lys
225                 230                 235                 240

Val His Glu Ile Gly Ile Ala Gly Lys Gln
                245                 250

<210>   4
<211>   644
<212>   PRT
<213>   Neisseria meningitidis

<400>   4
Met Ala Ser Pro Asp Val Lys Ser Ala Asp Thr Leu Ser Lys Pro Ala
1               5                   10                  15

Ala Pro Val Val Ser Glu Lys Glu Thr Glu Ala Lys Glu Asp Ala Pro
            20                  25                  30

Gln Ala Gly Ser Gln Gly Gln Gly Ala Pro Ser Ala Gln Gly Gly Gln
        35                  40                  45

Asp Met Ala Ala Val Ser Glu Glu Asn Thr Gly Asn Gly Gly Ala Ala
    50                  55                  60

Ala Thr Asp Lys Pro Lys Asn Glu Asp Glu Gly Ala Gln Asn Asp Met
65                  70                  75                  80

Pro Gln Asn Ala Ala Asp Thr Asp Ser Leu Thr Pro Asn His Thr Pro
                85                  90                  95

Ala Ser Asn Met Pro Ala Gly Asn Met Glu Asn Gln Ala Pro Asp Ala
            100                 105                 110

Gly Glu Ser Glu Gln Pro Ala Asn Gln Pro Asp Met Ala Asn Thr Ala
        115                 120                 125

Asp Gly Met Gln Gly Asp Asp Pro Ser Ala Gly Gly Glu Asn Ala Gly
    130                 135                 140

Asn Thr Ala Ala Gln Gly Thr Asn Gln Ala Glu Asn Asn Gln Thr Ala
145                 150                 155                 160

Gly Ser Gln Asn Pro Ala Ser Ser Thr Asn Pro Ser Ala Thr Asn Ser
                165                 170                 175

Gly Gly Asp Phe Gly Arg Thr Asn Val Gly Asn Ser Val Val Ile Asp
            180                 185                 190

Gly Pro Ser Gln Asn Ile Thr Leu Thr His Cys Lys Gly Asp Ser Cys
        195                 200                 205

Ser Gly Asn Asn Phe Leu Asp Glu Glu Val Gln Leu Lys Ser Glu Phe
    210                 215                 220

Glu Lys Leu Ser Asp Ala Asp Lys Ile Ser Asn Tyr Lys Lys Asp Gly
225                 230                 235                 240

Lys Asn Asp Gly Lys Asn Asp Lys Phe Val Gly Leu Val Ala Asp Ser
                245                 250                 255

Val Gln Met Lys Gly Ile Asn Gln Tyr Ile Ile Phe Tyr Lys Pro Lys
            260                 265                 270

Pro Thr Ser Phe Ala Arg Phe Arg Arg Ser Ala Arg Ser Arg Arg Ser
        275                 280                 285

Leu Pro Ala Glu Met Pro Leu Ile Pro Val Asn Gln Ala Asp Thr Leu
    290                 295                 300

Ile Val Asp Gly Glu Ala Val Ser Leu Thr Gly His Ser Gly Asn Ile
305                 310                 315                 320

Phe Ala Pro Glu Gly Asn Tyr Arg Tyr Leu Thr Tyr Gly Ala Glu Lys
                325                 330                 335

Leu Pro Gly Gly Ser Tyr Ala Leu Arg Val Gln Gly Glu Pro Ser Lys
            340                 345                 350

Gly Glu Met Leu Ala Gly Thr Ala Val Tyr Asn Gly Glu Val Leu His
        355                 360                 365

Phe His Thr Glu Asn Gly Arg Pro Ser Pro Ser Arg Gly Arg Phe Ala
    370                 375                 380

Ala Lys Val Asp Phe Gly Ser Lys Ser Val Asp Gly Ile Ile Asp Ser
385                 390                 395                 400

Gly Asp Gly Leu His Met Gly Thr Gln Lys Phe Lys Ala Ala Ile Asp
                405                 410                 415

Gly Asn Gly Phe Lys Gly Thr Trp Thr Glu Asn Gly Gly Gly Asp Val
            420                 425                 430

Ser Gly Lys Phe Tyr Gly Pro Ala Gly Glu Glu Val Ala Gly Lys Tyr
        435                 440                 445

Ser Tyr Arg Pro Thr Asp Ala Glu Lys Gly Gly Phe Gly Val Phe Ala
    450                 455                 460

Gly Lys Lys Glu Gln Asp Gly Ser Gly Gly Gly Gly Ala Thr Tyr Lys
465                 470                 475                 480

Val Asp Glu Tyr His Ala Asn Ala Arg Phe Ala Ile Asp His Phe Asn
                485                 490                 495

Thr Ser Thr Asn Val Gly Gly Phe Tyr Gly Leu Thr Gly Ser Val Glu
            500                 505                 510

Phe Asp Gln Ala Lys Arg Asp Gly Lys Ile Asp Ile Thr Ile Pro Val
        515                 520                 525

Ala Asn Leu Gln Ser Gly Ser Gln His Phe Thr Asp His Leu Lys Ser
    530                 535                 540

Ala Asp Ile Phe Asp Ala Ala Gln Tyr Pro Asp Ile Arg Phe Val Ser
545                 550                 555                 560

Thr Lys Phe Asn Phe Asn Gly Lys Lys Leu Val Ser Val Asp Gly Asn
                565                 570                 575

Leu Thr Met His Gly Lys Thr Ala Pro Val Lys Leu Lys Ala Glu Lys
            580                 585                 590

Phe Asn Cys Tyr Gln Ser Pro Met Ala Lys Thr Glu Val Cys Gly Gly
        595                 600                 605

Asp Phe Ser Thr Thr Ile Asp Arg Thr Lys Trp Gly Val Asp Tyr Leu
    610                 615                 620

Val Asn Val Gly Met Thr Lys Ser Val Arg Ile Asp Ile Gln Ile Glu
625                 630                 635                 640

Ala Ala Lys Gln


<210>  5
<211>  434
<212>  PRT
<213>  Neisseria meningitidis

<400>  5
Met Val Ser Ala Val Ile Gly Ser Ala Ala Val Gly Ala Lys Ser Ala
1               5                   10                  15

Val Asp Arg Arg Thr Thr Gly Ala Gln Thr Asp Asp Asn Val Met Ala
            20                  25                  30

Leu Arg Ile Glu Thr Thr Ala Arg Ser Tyr Leu Arg Gln Asn Asn Gln

35                  40                  45

Thr Lys Gly Tyr Thr Pro Gln Ile Ser Val Val Gly Tyr Asn Arg His
    50                  55                  60

Leu Leu Leu Leu Gly Gln Val Ala Thr Glu Gly Glu Lys Gln Phe Val
65                  70                  75                  80

Gly Gln Ile Ala Arg Ser Glu Gln Ala Ala Glu Gly Val Tyr Asn Tyr
                85                  90                  95

Ile Thr Val Ala Ser Leu Pro Arg Thr Ala Gly Asp Ile Ala Gly Asp
            100                 105                 110

Thr Trp Asn Thr Ser Lys Val Arg Ala Thr Leu Leu Gly Ile Ser Pro
        115                 120                 125

Ala Thr Gln Ala Arg Val Lys Ile Val Thr Tyr Gly Asn Val Thr Tyr
    130                 135                 140

Val Met Gly Ile Leu Thr Pro Glu Glu Gln Ala Gln Ile Thr Gln Lys
145                 150                 155                 160

Val Ser Thr Thr Val Gly Val Gln Lys Val Ile Thr Leu Tyr Gln Asn
                165                 170                 175

Tyr Val Gln Arg Gly Ser Gly Gly Gly Gly Val Ala Ala Asp Ile Gly
            180                 185                 190

Ala Gly Leu Ala Asp Ala Leu Thr Ala Pro Leu Asp His Lys Asp Lys
        195                 200                 205

Gly Leu Gln Ser Leu Thr Leu Asp Gln Ser Val Arg Lys Asn Glu Lys
    210                 215                 220

Leu Lys Leu Ala Ala Gln Gly Ala Glu Lys Thr Tyr Gly Asn Gly Asp
225                 230                 235                 240

Ser Leu Asn Thr Gly Lys Leu Lys Asn Asp Lys Val Ser Arg Phe Asp
                245                 250                 255

Phe Ile Arg Gln Ile Glu Val Asp Gly Gln Leu Ile Thr Leu Glu Ser
            260                 265                 270

Gly Glu Phe Gln Val Tyr Lys Gln Ser His Ser Ala Leu Thr Ala Phe
        275                 280                 285

Gln Thr Glu Gln Ile Gln Asp Ser Glu His Ser Gly Lys Met Val Ala
    290                 295                 300

Lys Arg Gln Phe Arg Ile Gly Asp Ile Ala Gly Glu His Thr Ser Phe
305                 310                 315                 320

Asp Lys Leu Pro Glu Gly Gly Arg Ala Thr Tyr Arg Gly Thr Ala Phe
                325                 330                 335

Gly Ser Asp Asp Ala Gly Gly Lys Leu Thr Tyr Thr Ile Asp Phe Ala
            340                 345                 350

Ala Lys Gln Gly Asn Gly Lys Ile Glu His Leu Lys Ser Pro Glu Leu
        355                 360                 365

Asn Val Asp Leu Ala Ala Ala Asp Ile Lys Pro Asp Gly Lys Arg His
    370                 375                 380

Ala Val Ile Ser Gly Ser Val Leu Tyr Asn Gln Ala Glu Lys Gly Ser
385                 390                 395                 400

Tyr Ser Leu Gly Ile Phe Gly Gly Lys Ala Gln Glu Val Ala Gly Ser
                405                 410                 415

Ala Glu Val Lys Thr Val Asn Gly Ile Arg His Ile Gly Leu Ala Ala
            420                 425                 430

Lys Gln

<210>   6
<211>   327
<212>   PRT
<213>   Neisseria meningitidis

<400>   6
Ala Thr Asn Asp Asp Asp Val Lys Lys Ala Ala Thr Val Ala Ile Ala
1               5                   10                  15

Ala Ala Tyr Asn Asn Gly Gln Glu Ile Asn Gly Phe Lys Ala Gly Glu
            20                  25                  30

Thr Ile Tyr Asp Ile Asp Glu Asp Gly Thr Ile Thr Lys Lys Asp Ala
        35                  40                  45

Thr Ala Ala Asp Val Glu Ala Asp Asp Phe Lys Gly Leu Gly Leu Lys
    50                  55                  60

Lys Val Val Thr Asn Leu Thr Lys Thr Val Asn Glu Asn Lys Gln Asn
65                  70                  75                  80

Val Asp Ala Lys Val Lys Ala Ala Glu Ser Glu Ile Glu Lys Leu Thr
                85                  90                  95

Thr Lys Leu Ala Asp Thr Asp Ala Ala Leu Ala Asp Thr Asp Ala Ala
            100                 105                 110

Leu Asp Ala Thr Thr Asn Ala Leu Asn Lys Leu Gly Glu Asn Ile Thr
        115                 120                 125

Thr Phe Ala Glu Glu Thr Lys Thr Asn Ile Val Lys Ile Asp Glu Lys
    130                 135                 140

Leu Glu Ala Val Ala Asp Thr Val Asp Lys His Ala Glu Ala Phe Asn
145                 150                 155                 160

Asp Ile Ala Asp Ser Leu Asp Glu Thr Asn Thr Lys Ala Asp Glu Ala
                165                 170                 175

Val Lys Thr Ala Asn Glu Ala Lys Gln Thr Ala Glu Glu Thr Lys Gln
            180                 185                 190

Asn Val Asp Ala Lys Val Lys Ala Ala Glu Thr Ala Ala Gly Lys Ala
        195                 200                 205

Glu Ala Ala Ala Gly Thr Ala Asn Thr Ala Ala Asp Lys Ala Glu Ala
    210                 215                 220

Val Ala Ala Lys Val Thr Asp Ile Lys Ala Asp Ile Ala Thr Asn Lys
225                 230                 235                 240

Asp Asn Ile Ala Lys Lys Ala Asn Ser Ala Asp Val Tyr Thr Arg Glu
                245                 250                 255

Glu Ser Asp Ser Lys Phe Val Arg Ile Asp Gly Leu Asn Ala Thr Thr
            260                 265                 270

Glu Lys Leu Asp Thr Arg Leu Ala Ser Ala Glu Lys Ser Ile Ala Asp
        275                 280                 285

His Asp Thr Arg Leu Asn Gly Leu Asp Lys Thr Val Ser Asp Leu Arg
    290                 295                 300

Lys Glu Thr Arg Gln Gly Leu Ala Glu Gln Ala Ala Leu Ser Gly Leu
305                 310                 315                 320

Phe Gln Pro Tyr Asn Val Gly
                325

<210>   7
<211>   434
<212>   PRT
<213>   Neisseria meningitidis

<400>   7
Met Val Ser Ala Val Ile Gly Ser Ala Ala Val Gly Ala Lys Ser Ala
1               5                   10                  15

Val Asp Arg Arg Thr Thr Gly Ala Gln Thr Asp Asp Asn Val Met Ala
            20                  25                  30

Leu Arg Ile Glu Thr Thr Ala Arg Ser Tyr Leu Arg Gln Asn Asn Gln
        35                  40                  45

Thr Lys Gly Tyr Thr Pro Gln Ile Ser Val Val Gly Tyr Asp Arg His
    50                  55                  60

Leu Leu Leu Leu Gly Gln Val Ala Thr Glu Gly Glu Lys Gln Phe Val
65                  70                  75                  80

Gly Gln Ile Ala Arg Ser Glu Gln Ala Ala Glu Gly Val Tyr Asn Tyr
                85                  90                  95

Ile Thr Val Ala Ser Leu Pro Arg Thr Ala Gly Asp Ile Ala Gly Asp
            100                 105                 110

Thr Trp Asn Thr Ser Lys Val Arg Ala Thr Leu Leu Gly Ile Ser Pro
        115                 120                 125

Ala Thr Arg Ala Arg Val Lys Ile Val Thr Tyr Gly Asn Val Thr Tyr
    130                 135                 140

Val Met Gly Ile Leu Thr Pro Glu Glu Gln Ala Gln Ile Thr Gln Lys
145                 150                 155                 160

Val Ser Thr Thr Val Gly Val Gln Lys Val Ile Thr Leu Tyr Gln Asn
                165                 170                 175

Tyr Val Gln Arg Gly Ser Gly Gly Gly Gly Val Ala Ala Asp Ile Gly
            180                 185                 190

Ala Gly Leu Ala Asp Ala Leu Thr Ala Pro Leu Asp His Lys Asp Lys
        195                 200                 205

Gly Leu Gln Ser Leu Thr Leu Asp Gln Ser Val Arg Lys Asn Glu Lys
    210                 215                 220

Leu Lys Leu Ala Ala Gln Gly Ala Glu Lys Thr Tyr Gly Asn Gly Asp

225                 230                 235                 240

Ser Leu Asn Thr Gly Lys Leu Lys Asn Asp Lys Val Ser Arg Phe Asp
                245                 250                 255

Phe Ile Arg Gln Ile Glu Val Asp Gly Gln Leu Ile Thr Leu Glu Ser
            260                 265                 270

Gly Glu Phe Gln Val Tyr Lys Gln Ser His Ser Ala Leu Thr Ala Phe
        275                 280                 285

Gln Thr Glu Gln Ile Gln Asp Ser Glu His Ser Gly Lys Met Val Ala
    290                 295                 300

Lys Arg Gln Phe Arg Ile Gly Asp Ile Ala Gly Glu His Thr Ser Phe
305                 310                 315                 320

Asp Lys Leu Pro Glu Gly Gly Arg Ala Thr Tyr Arg Gly Thr Ala Phe
                325                 330                 335

Gly Ser Asp Asp Ala Gly Gly Lys Leu Thr Tyr Thr Ile Asp Phe Ala
            340                 345                 350

Ala Lys Gln Gly Asn Gly Lys Ile Glu His Leu Lys Ser Pro Glu Leu
        355                 360                 365

Asn Val Asp Leu Ala Ala Ala Asp Ile Lys Pro Asp Gly Lys Arg His
    370                 375                 380

Ala Val Ile Ser Gly Ser Val Leu Tyr Asn Gln Ala Glu Lys Gly Ser
385                 390                 395                 400

Tyr Ser Leu Gly Ile Phe Gly Gly Lys Ala Gln Glu Val Ala Gly Ser
                405                 410                 415

Ala Glu Val Lys Thr Val Asn Gly Ile Arg His Ile Gly Leu Ala Ala
            420                 425                 430

Lys Gln

## Description

### TECHNICAL FIELD

This invention is in the field of formulating meningococcal vaccines.

### BACKGROUND ART

Various vaccines against serogroup B of *Neisseria meningitidis* ("Men-B") are currently being investigated, but they share one common characteristic.

The outer membrane vesicle (OMV) products produced by Novartis (MENZB™), by the Finlay Institute (VA-MENGOC-BC™), by the Norwegian Institute of Public Health (MENBVAC™) and by the Netherlands Vaccine Institute (HEXAMEN™ and NONAMEN™) all include an aluminium hydroxide adjuvant. The "universal vaccine for serogroup B meningococcus" reported by Novartis in reference 1 also includes an aluminium hydroxide adjuvant, as did the bivalent OMV vaccine recently reported in reference 2.

### DISCLOSURE OF THE INVENTION

In developing the Novartis Men-B vaccine, the inventors have found that optimum immunogenicity requires the presence of an adjuvant. Moreover, they have found that adsorption to aluminium hydroxide provides good storage stability for the vaccine antigens. To avoid the need to use aluminium salts, however, alternatives have been sought.

The use of non-aluminium adjuvants for Men-B vaccines is already known. For instance, reference 3 reports the use of the MF59 oil-in-water emulsion as an adjuvant for Men-B vesicles, and reference 4 describes the use of immunostimulatory oligonucleotides and/or MF59 as adjuvants. Although the immunogenicity results with MF59 were excellent, and continued research has shown that this adjuvant can enhance the strain coverage of a Men-B vaccine when compared to aluminium hydroxide, further work has unexpectedly shown that Men-B immunogens adjuvanted with oil-in-water emulsions have poor long-term stability. Thus it is an object of the invention to provide ways of improving the storage stability of Men-B vaccines when using oil-in-water emulsion adjuvants.

Previous experience with oil-in-water emulsions comes from the area of influenza vaccines. The FLUAD™ product includes the MF59 emulsion, and it is distributed in a pre-mixed liquid format in a single container (the 'one vial' approach). This pre-mixed formulation is the formulation that has now been found to offer poor stability for Men-B vaccines.

As an alternative to a 'one vial' formulation with oil-in-water emulsion vaccines, a 'two vial' approach has been used for influenza (e.g. see ref. 5), for HSV (e.g. ref. 6) and for HIV *(e.g.* ref. 7), in which the vaccine and emulsion are distributed together, both in liquid format, for extemporaneous mixing at the point of use.

According to the present invention, a different approach is followed for Men-B vaccines, using a dual formulation of (i) an oil-in-water emulsion adjuvant and (ii) a Men-B immunogenic component in lyophilised form. The lyophilised Men-B antigens can be reconstituted into liquid adjuvanted form at the time of use ready for administration to a patient. This formulation has been found to give excellent result in terms of both stability and immunogenicity.

The inventors have further found that the lyophilised component can retain efficacy when one or more conjugated saccharides from *N.meningitidis* in serogroups A, C, W135 and/or Y (Men-A, -C, -W135 and -Y) is included. The combination of antigens for immunising against multiple meningococcal serogroups, including serogroup B, using a single lyophilised component is particularly advantageous.

Thus the invention provides a kit comprising: (i) a first container containing an adjuvant comprising an oil-in-water emulsion; and (ii) a second container containing a lyophilised antigenic composition comprising an immunogen for raising an immune response against *N.meningitidis* serogroup B. The lyophilised antigenic composition may further comprise a conjugated capsular saccharide from one or more *of N.meningitidis* serogroups A, C, W135 and/or Y.

The invention also provides a method for preparing an immunogenic composition, comprising a step of mixing: (i) an adjuvant comprising an oil-in-water emulsion; and (ii) a lyophilised antigenic composition comprising an immunogen for raising an immune response against *N.meningitidis* serogroup B. The lyophilised antigenic composition may further comprise a conjugated capsular saccharide from one or more *of N.meningitidis* serogroups A, C, W135 and/or Y.

The invention also provides a lyophilised antigenic composition comprising (i) an immunogen for raising an immune response against *N.meningitidis* serogroup B, and (ii) a conjugated capsular saccharide from one or more of *N.meningitidis* serogroups A, C, W135 and/or Y. This lyophilised composition is suitable for reconstitution by an adjuvant comprising an oil-in-water emulsion, and is suitable for use as a kit component of the invention.

### The lyophilised antigenic composition

The invention uses a lyophilised antigenic composition that includes an immunogen for raising an immune response against Men-B. It may optionally include a conjugated capsular saccharide from one or more of Men-A, Men-C, Men-W135 and/or Men-Y.

The Men-B immunogen may comprise membrane vesicles from a Men-B bacterium and/or Men-B recombinant proteins and/or Men-B lipo-oligosaccharide (LOS).

Lyophilisation of Men-B OMVs is known in the art [8], but these OMVs were subsequently administered together with an aluminium phosphate adjuvant. Thus The problem of antigen stability when combined with an oil-in-water emulsion adjuvant was not reported. Similarly lyophilisation of meningococcal conjugate antigens is known, including subsequent reconstitution with MF59 [9], but not in combination with any Men-B antigen.

### Men-B components comprising vesicles

Vesicles for use as Men-B vaccine components include any proteoliposomic vesicle obtained by disrupting a bacterial outer membrane to form vesicles therefrom that include protein components of the outer membrane. Thus the term includes OMVs (sometimes referred to as 'blebs'), microvesicles (MVs [10]) and 'native OMVs' ('NOMVs' [11]).

MVs and NOMVs are naturally-occurring membrane vesicles that form spontaneously during bacterial growth and are released into culture medium. MVs can be obtained by culturing *Neisseria* in broth culture medium, separating whole cells from the smaller MVs in the broth culture medium (e.g. by filtration or by low-speed centrifugation to pellet only the cells and not the smaller vesicles), and then collecting the MVs from the cell-depleted medium (e.g. by filtration, by differential precipitation or aggregation of MVs, by high-speed centrifugation to pellet the MVs). Strains for use in production of MVs can generally be selected on the basis of the amount of MVs produced in culture e.g. refs. 12 & 13 describe *Neisseria* with high MV production.

OMVs are prepared artificially from bacteria, and may be prepared using detergent treatment (e.g. with deoxycholate), or by non-detergent means (e.g. see reference 14). Methods for obtaining suitable OMV preparations are disclosed in, for instance, the references cited herein. Techniques for forming OMVs include treating bacteria with a bile acid salt detergent (e.g. salts of lithocholic acid, chenodeoxycholic acid, ursodeoxycholic acid, deoxycholic acid, cholic acid, ursocholic acid, *etc.,* with sodium deoxycholate [15 & 16] being preferred for treating *Neisseria*) at a pH sufficiently high not to precipitate the detergent [17]. Other techniques may be performed substantially in the absence of detergent [14] using techniques such as sonication, homogenisation, microfluidisation, cavitation, osmotic shock, grinding, French press, blending, *etc.* Methods using no or low detergent can retain useful antigens such as NspA [14]. Thus a method may use an OMV extraction buffer with about 0.5% deoxycholate or lower e.g. about 0.2%, about 0.1%, <0.05% or zero.

A useful process for OMV preparation is described in reference 18 and involves ultrafiltration on crude OMVs, rather than instead of high speed centrifugation. The process may involve a step of ultracentrifugation after the ultrafiltration takes place.

Vesicles an be prepared from any Men-B strain for use with the invention. They may be of any serotype *(e.g.* 1, 2a, 2b, 4, 14, 15, 16, *etc.),* any serosubtype, and any immunotype (e.g. L1; L2; L3; L3,3,7; L10; *etc.).* The meningococci may be from any suitable lineage, including hyperinvasive and hypervirulent lineages e.g. any of the following seven hypervirulent lineages: subgroup I; subgroup III; subgroup IV-1; ET-5 complex; ET-37 complex; A4 cluster; lineage 3. These lineages have been defined by multilocus enzyme electrophoresis (MLEE), but multilocus sequence typing (MLST) has also been used to classify meningococci [ref. 19] e.g. the ET-37 complex is the ST-11 complex by MLST, the ET-5 complex is ST-32 (ET-5), lineage 3 is ST-41/44, *etc.* Vesicles can be prepared from strains having one of the following subtypes: P1.2; P1.2,5; P1.4; P1.5; P1.5,2; P1.5,c; P1.5c,10; P1.7,16; P1.7,16b; P1.7h,4; P1.9; P1.15; P1.9,15; P1.12,13; P1.13; P1.14; P1.21,16; P1.22,14.

Vesicles used with the invention may be prepared from wild-type Men-B strains or from mutant strains. For instance, reference 20 discloses preparations of vesicles obtained from *N.meningitidis* with a modified *fur* gene. Reference 27 teaches that *nspA* expression should be up-regulated with concomitant *porA* and *cps* knockout. Further knockout mutants of *N.meningitidis* for OMV production are disclosed in references 27 to 29. Reference 21 discloses vesicles in which fHBP is upregulated. Reference 22 discloses the construction of vesicles from strains modified to express six different PorA subtypes. Mutant *Neisseria* with low endotoxin levels, achieved by knockout of enzymes involved in LPS biosynthesis, may also be used [23,24]. These or others mutants can all be used with the invention.

Thus a Men-B strain used with the invention may in some embodiments express more than one PorA subtype. 6-valent and 9-valent PorA strains have previously been constructed. The strain may express 2, 3, 4, 5, 6, 7, 8 or 9 of PorA subtypes: P1.7,16; P1.5-1,2-2; P1.19,15-1; P1.5-2,10; P1.12-1,13; P1.7-2,4; P1.22,14; P1.7-1,1 and/or P1.18-1,3,6. In other embodiments a strain may have been down-regulated for PorA expression e.g. in which the amount of PorA has been reduced by at least 20% (e.g. ≥30%, ≥40%, ≥50%, ≥60%, ≥70%, ≥80%, ≥90%, ≥95%, *etc.),* or even knocked out, relative to wild-type levels (e.g. relative to strain H44/76, as disclosed in reference 30).

In some embodiments a Men-B strain may over-express (relative to the corresponding wild-type strain) certain proteins. For instance, strains may over-express NspA, protein 287 [45], fHBP [21], TbpA and/or TbpB [25], Cu,Zn-superoxide dismutase [25], *etc.*

In some embodiments a Men-B strain may include one or more of the knockout and/or over-expression mutations disclosed in references 26 to 29. Preferred genes for down-regulation and/or knockout include: (a) Cps, CtrA, CtrB, CtrC, CtrD, FrpB, GalE, HtrB/MsbB, LbpA, LbpB, LpxK, Opa, Opc, PilC, PorB, SiaA, SiaB, SiaC, SiaD, TbpA, and/or TbpB [26]; (b) CtrA, CtrB, CtrC, CtrD, FrpB, GalE, HtrB/MsbB, LbpA, LbpB, LpxK, Opa, Opc, PhoP, PilC, PmrE, PmrF, SiaA, SiaB, SiaC, SiaD, TbpA, and/or TbpB [27]; (c) ExbB, ExbD, rmpM, CtrA, CtrB, CtrD, GalE, LbpA, LpbB, Opa, Opc, PilC, PorB, SiaA, SiaB, SiaC, SiaD, TbpA, and/or TbpB [28]; and (d) CtrA, CtrB, CtrD, FrpB, OpA, OpC, PilC, PorB, SiaD, SynA, SynB, and/or SynC [29].

Where a mutant strain is used, in some embodiments it may have one or more, or all, of the following characteristics: (i) down-regulated or knocked-out LgtB and/or GalE to truncate the meningococcal LOS; (ii) up-regulated TbpA; (iii) up-regulated Hsf; (iv) up-regulated Omp85; (v) up-regulated LbpA; (vi) up-regulated NspA; (vii) knocked-out PorA; (viii) down-regulated or knocked-out FrpB; (ix) down-regulated or knocked-out Opa; (x) down-regulated or knocked-out Opc; (xii) deleted *cps* gene complex. A truncated LOS can be one that does not include a sialyl-lacto-N-neotetraose epitope e.g. it might be a galactose-deficient LOS. The LOS may have no α chain.

If LOS is present in a vesicle then it is possible to treat the vesicle so as to link its LOS and protein components ("intra-bleb" conjugation [29]).

The invention may be used with mixtures of vesicles from different strains. For instance, reference 30 discloses vaccine comprising multivalent meningococcal vesicle compositions, comprising a first vesicle derived from a meningococcal strain with a serosubtype prevalent in a country of use, and a second vesicle derived from a strain that need not have a serosubtype prevent in a country of use. Reference 31 also discloses useful combinations of different vesicles. A combination of vesicles from strains in each of the L2 and L3 immunotypes may be used in some embodiments.

Vesicle-based antigens can be prepared from serogroups other than Men-B (e.g. reference 17 discloses a process for Men-A). The invention may accordingly be used with vesicles prepared serogroups other than Men-B (e.g. A, C, W135 and/or Y). The main focus, however, is on Men-B.

### Men-B components comprising recombinant proteins

Recombinant proteins have also been reported for use as vaccine immunogens against Men-B. For instance, various antigens are reported in references 32 to 40. Such antigens may be used alone or in combinations. Where multiple purified proteins are combined then it is helpful to use a mixture of 10 or fewer (e.g. 9, 8, 7, 6, 5, 4, 3, 2) purified antigens.

A particularly useful combination of antigens is disclosed in references 1 and 40, and so a composition of the invention may include 1, 2, 3, 4 or 5 of: (1) a 'NadA' protein; (2) a 'fHBP' protein, formerly known as '741'; (3) a '936' protein; (4) a '953' protein; and (5) a '287' protein. Other possible antigen combinations may comprise a transferrin binding protein (e.g. TbpA and/or TbpB) and a Hsf antigen. Other possible purified antigens include proteins comprising one of the following amino acid sequences: SEQ ID NO:650 from ref. 32; SEQ ID NO:878 from ref. 32; SEQ ID NO:884 from ref. 32; SEQ ID NO:4 from ref. 33; SEQ ID NO:598 from ref. 34; SEQ ID NO:818 from ref. 34; SEQ ID NO:864 from ref. 34; SEQ ID NO:866 from ref. 34; SEQ ID NO:1196 from ref. 34; SEQ ID NO:1272 from ref. 34; SEQ ID NO:1274 from ref. 34; SEQ ID NO:1640 from ref. 34; SEQ ID NO:1788 from ref. 34; SEQ ID NO:2288 from ref. 34; SEQ ID NO:2466 from ref. 34; SEQ ID NO:2554 from ref. 34; SEQ ID NO:2576 from ref. 34; SEQ ID NO:2606 from ref. 34; SEQ ID NO:2608 from ref. 34; SEQ ID NO:2616 from ref. 34; SEQ ID NO:2668 from ref. 34; SEQ ID NO:2780 from ref. 34; SEQ ID NO:2932 from ref. 34; SEQ ID NO:2958 from ref. 34; SEQ ID NO:2970 from ref. 34; SEQ ID NO:2988 from ref. 34, or a polypeptide comprising an amino acid sequence which: (a) has 50% or more identity (e.g. 60%, 70%, 80%, 90%, 95%, 99% or more) to said sequences; and/or (b) comprises a fragment of at least n consecutive amino acids from said sequences, wherein n is 7 or more (*eg.* 8, 10, 12, 14, 16, 18, 20, 25, 30, 35, 40, 50, 60, 70, 80, 90, 100, 150, 200, 250 or more). Preferred fragments for (b) comprise an epitope from the relevant sequence. More than one (e.g. 2, 3, 4, 5, 6) of these polypeptides may be included.

The fHBP antigen falls into three distinct variants [39]. A Men-B component of the invention may include a single fHBP variant, but is will usefully include a fHBP from each of two or all three variants. Thus it may include a combination of two or three different purified fHBPs, selected from: (a) a first protein, comprising an amino acid sequence having at least *a%* sequence identity to SEQ ID NO: 1 and/or comprising an amino acid sequence consisting of a fragment of at least x contiguous amino acids from SEQ ID NO: 1; (b) a second protein, comprising an amino acid sequence having at least *b%* sequence identity to SEQ ID NO: 2 and/or comprising an amino acid sequence consisting of a fragment of at least *y* contiguous amino acids from SEQ ID NO: 2; and/or (c) a third protein, comprising an amino acid sequence having at least c% sequence identity to SEQ ID NO: 3 and/or comprising an amino acid sequence consisting of a fragment of at least z contiguous amino acids from SEQ ID NO: 3.

The value of *a* is at least 85 *e.g.* 86, 87, 88, 89, 90, 91, 92, 93, 94, 95, 96, 97, 98, 99, 99.5, or more. The value of *b* is at least 85 *e.g.* 86, 87, 88, 89, 90, 91, 92, 93, 94, 95, 96, 97, 98, 99, 99.5, or more. The value of c is at least 85 e.g. 86, 87, 88, 89, 90, 91, 92, 93, 94, 95, 96, 97, 98, 99, 99.5, or more. The values of *a, b* and c are not intrinsically related to each other.

The value of x is at least 7 e.g. 8, 9, 10, 11, 12, 13, 14, 15, 16, 17, 18, 19, 20, 21, 22, 23, 24, 25, 26, 27, 28, 29, 30, 35, 40, 45, 50, 60, 70, 80, 90, 100, 120, 140, 160, 180, 200, 225, 250). The value of *y* is at least 7 e.g. 8, 9, 10, 11, 12, 13, 14, 15, 16, 17, 18, 19, 20, 21, 22, 23, 24, 25, 26, 27, 28, 29, 30, 35, 40, 45, 50, 60, 70, 80, 90, 100, 120, 140, 160, 180, 200, 225, 250). The value of z is at least 7 e.g. 8, 9, 10, 11, 12, 13, 14, 15, 16, 17, 18, 19, 20, 21, 22, 23, 24, 25, 26, 27, 28, 29, 30, 35, 40, 45, 50, 60, 70, 80, 90, 100, 120, 140, 160, 180, 200, 225, 250). The values of x, y and z are not intrinsically related to each other.

In some embodiments fHBP protein(s) will be lipidated e.g. at a N-terminus cysteine. In other embodiments they will not be lipidated.

A useful composition based on purified proteins comprises a mixture of: (i) a first polypeptide having amino acid sequence SEQ ID NO: 4; (ii) a first polypeptide having amino acid sequence SEQ ID NO: 5 or SEQ ID NO: 7; and (iii) a first polypeptide having amino acid sequence SEQ ID NO: 6. See refs. 1 & 40.

### Men-B components comprising LOS

Meningococcal vaccines based on lipooligosaccharide have been reported. The LOS may be used on its own or conjugated to a carrier. When it is conjugated, conjugation may be via a lipid A portion in the LOS or by any other suitable moiety *e.g.* its KDO residues. If the lipid A moiety of LOS is absent then such alternative linking is essential.

The LOS may be from any immunotype *e.g.* L2, L3, L7, *etc.*

Rather than use native LOS, it is preferred to use a modified form. These modifications can be achieved chemically, but it is more convenient to knockout the enzymes in Men-B responsible for certain biosynthetic additions. For instance, LOS may be modified to remove at least the terminal Gal of the native lacto-N-neotetraose unit, and this modification can be achieved by knocking out one or more of the relevant enzymes. The enzymes responsible for adding the two terminal monosaccharides in a native LOS (sialic acid and galactose) can be knocked out, either to eliminate just the terminal Sia or to eliminate the Sia-Gal disaccharide. Knocking out the *IgtB* gene, for instance, removes Sia-Gal. A knockout of the *galE* gene also provides a useful modified LOS. A lipid A fatty transferase gene may be knocked out [41].

At least one primary O-linked fatty acid may be removed from LOS [42]. LOS having a reduced number of secondary acyl chains per LOS molecule can also be used [43]. The LOS may have no α chain.

The LOS may comprise GlcNAc-Hep₂phosphoethanolamine-KDO₂-Lipid A [44].

### Mixed Men-B components

The invention may use vesicles, purified polypeptides or LOS as the Men-B antigen. It may also use combinations of these three antigens e.g. (i) vesicles + purified polypeptides; (ii) vesicles + LOS; (iii) purified polypeptides + LOS; or (iv) vesicles + purified polypeptides + LOS. These combinations may be made by preparing the individual components separately and then mixing them. For instance, reference 45 discloses adding purified proteins to vesicles to provide a composition with broader efficacy.

### Serogroups A, C, W135 and Y

Conjugated monovalent vaccines against serogroup C have been approved for human use, and include MENJUGATE™, MENINGITEC™ and NEISVAC-C™. Mixtures of conjugates from serogroups A+C are known [46,47] and mixtures of conjugates from serogroups A+C+W135+Y have been reported [48-51] and were approved in 2005 as the aqueous MENACTRA™ product.

The lyophilised component used with the invention may include one or more conjugates of capsular saccharides from 1, 2, 3, or 4 of meningococcal serogroups A, C, W135 and Y e.g. A+C, A+W135, A+Y, C+W135, C+Y, W135+Y, A+C+W135, A+C+Y, A+W135+Y, A+C+W135+Y, *etc.* Components including saccharides from all four of serogroups A, C, W135 and Y are preferred.

The capsular saccharide of serogroup A meningococcus is a homopolymer of (α1→6)-linked N-acetyl-D-mannosamine-1-phosphate, with partial O-acetylation in the C3 and C4 positions. Acetylation at the C-3 position can be 70-95%. Conditions used to purify the saccharide can result in de-O-acetylation (e.g. under basic conditions), but it is useful to retain OAc at this C-3 position. In some embodiments, at least 50% *(e.g.* at least 60%, 70%, 80%, 90%, 95% or more) of the mannosamine residues in a serogroup A saccharides are O-acetylated at the C-3 position. Acetyl groups can be replaced with blocking groups to prevent hydrolysis [52], and such modified saccharides are still serogroup A saccharides within the meaning of the invention.

The serogroup C capsular saccharide is a homopolymer of (α 2→9)-linked sialic acid (N-acetyl neuraminic acid, or 'NeuNAc'). The saccharide structure is written as →9)-Neu p NAc 7/8 OAc-(α2→. Most serogroup C strains have O-acetyl groups at C-7 and/or C-8 of the sialic acid residues, but about 15% of clinical isolates lack these O-acetyl groups [53,54].The presence or absence of OAc groups generates unique epitopes, and the specificity of antibody binding to the saccharide may affect its bactericidal activity against O-acetylated (OAc+) and de-O-acetylated (OAc-) strains [55-57]. Serogroup C saccharides used with the invention may be prepared from either OAc+ or OAc-strains. Licensed Men-C conjugate vaccines include both OAc- (NEISVAC-C™) and OAc+ (MENJUGATE™ & MENINGITEC™) saccharides. In some embodiments, strains for production of serogroup C conjugates are OAc+ strains, e.g. of serotype 16, serosubtype P1.7a,1, *etc..* Thus C:16:P1.7a,1 OAc+ strains may be used. OAc+ strains in serosubtype P1.1 are also useful, such as the C11 strain.

The serogroup W135 saccharide is a polymer of sialic acid-galactose disaccharide units. Like the serogroup C saccharide, it has variable O-acetylation, but at sialic acid 7 and 9 positions [58]. The structure is written as: →4)-D-Neu*p*5Ac(7/9OAc)-α-(2→6)-D-Gal-α-(1→.

The serogroup Y saccharide is similar to the serogroup W135 saccharide, except that the disaccharide repeating unit includes glucose instead of galactose. Like serogroup W135, it has variable O-acetylation at sialic acid 7 and 9 positions [58]. The serogroup Y structure is written as: →4)-D-Neu*p*5Ac(7/9OAc)-α-(2→6)-D-Glc-α-(1→.

The saccharides used according to the invention may be O-acetylated as described above (e.g. with the same O-acetylation pattern as seen in native capsular saccharides), or they may be partially or totally de-O-acetylated at one or more positions of the saccharide rings, or they may be hyper-O-acetylated relative to the native capsular saccharides.

The saccharide moieties in conjugates may comprise full-length saccharides as prepared from meningococci, and/or may comprise fragments of full-length saccharides i. e. the saccharides may be shorter than the native capsular saccharides seen in bacteria. The saccharides may thus be depolymerised, with depolymerisation occurring during or after saccharide purification but before conjugation. Depolymerisation reduces the chain length of the saccharides. One depolymerisation method involves the use of hydrogen peroxide [48]. Hydrogen peroxide is added to a saccharide (e.g. to give a final H₂O₂ concentration of 1%), and the mixture is then incubated (e.g. at about 55°C) until a desired chain length reduction has been achieved. Another depolymerisation method involves acid hydrolysis [49]. Other depolymerisation methods are known in the art. The saccharides used to prepare conjugates for use according to the invention may be obtainable by any of these depolymerisation methods. Depolymerisation can be used in order to provide an optimum chain length for immunogenicity and/or to reduce chain length for physical manageability of the saccharides. In some embodiments, saccharides have the following range of average degrees of polymerisation (Dp): A=10-20; C=12-22; W135=15-25; Y=15-25. In terms of molecular weight, rather than Dp, useful ranges are, for all serogroups: <100kDa; 5kDa-75kDa; 7kDa-50kDa; 8kDa-35kDa; 12kDa-25kDa; 15kDa-22kDa.

In some embodiments, the average molecular weight for saccharides from each of meningococcal serogroups A, C, W135 and Y may be more than 50kDa *e.g.* ≥75kDa, ≥100kDa, ≥110kDa, ≥120kDa, ≥130kDa, *etc.* [59], and even up to 1500kDa, in particular as determined by MALLS. For instance: a Men-A saccharide may be in the range 50-500kDa *e.g*.60-80kDa; a Men-C saccharide may be in the range 100-210kDa; a Men-W135 saccharide may be in the range 60-190kDa e.g.120-140kDa; and/or a Men-Y saccharide may be in the range 60-190kDa e.g.150-160kDa.

The mass of meningococcal saccharide per serogroup in the reconstituted vaccine will usually be between Iμg and 20μg e.g. between 2 and 10 μg per serogroup, or about 4μg or about 5μg or about 10μg. Where conjugates from more than one serogroup are included then they may be present at substantially equal masses e.g. the mass of each serogroup's saccharide is within +10% of each other. As an alternative to an equal ratio, a double mass of serogroup A saccharide may be used. Thus a vaccine may include Men-A saccharide at 10μg and Men-C, -W135 and -Y saccharides at 5μg each.

Preferred carrier proteins are bacterial toxins, such as diphtheria or tetanus toxins, or toxoids or mutants thereof. These are commonly used in conjugate vaccines. The CRM₁₉₇ diphtheria toxin mutant is particularly preferred [60]. Other suitable carrier proteins include the *N.meningitidis* outer membrane protein complex [61], synthetic peptides [62,63], heat shock proteins [64,65], pertussis proteins [66,67], cytokines [68], lymphokines [68], hormones [68], growth factors [68], artificial proteins comprising multiple human CD4⁺ T cell epitopes from various pathogen-derived antigens [69] such as N19 [70], protein D from *H.influenzae* [71-73], pneumolysin [74] or its non-toxic derivatives [75], pneumococcal surface protein PspA [76], iron-uptake proteins [77], toxin A or B from *C.difficile* [78], recombinant *Pseudomonas aeruginosa* exoprotein A (rEPA) [79], *etc.* A single carrier protein may carry saccharides from multiple different serogroups [80], but this arrangement is not preferred. Where the lyophilised component includes conjugates from more than one meningococcal serogroup then the various conjugates may use different carrier proteins (e.g. one serogroup on CRM197, another on tetanus toxoid) or they may use the same carrier protein (e.g. saccharides from two serogroups separately conjugated to CRM197 and then combined).

Conjugates with a saccharide:protein ratio (w/w) of between 1:5 (*i.e.* excess protein) and 5:1 (*i.e.* excess saccharide) may be used e.g. ratios between 1:2 and 5:1 and ratios between 1:1.25 and 1:2.5. As described in reference 81, different meningococcal serogroup conjugates in a mixture can have different saccharide:protein ratios e.g. one may have a ratio of between 1:2 & 1:5, whereas another has a ratio between 5:1 & 1:1.99.

Saccharides and conjugates having the characteristics disclosed in reference 82 are useful.

The carrier molecule may be covalently conjugated to the meningococcal saccharide directly or via a linker. Various linkers are known e.g. an adipic acid linker, which may be formed by coupling a free -NH₂ group (e.g. introduced to a saccharide by amination) with adipic acid (using, for example, diimide activation), and then coupling a protein to the resulting saccharide-adipic acid intermediate [83, 84]. Another preferred type of linkage is a carbonyl linker, which may be formed by reaction of a free hydroxyl group of a saccharide with CDI [85, 86] followed by reaction with a protein to form a carbamate linkage. Other linkers include β-propionamido [87], nitrophenyl-ethylamine [88], haloacyl halides [89], glycosidic linkages [90], 6-aminocaproic acid [91], N-succinimidyl-3-(2-pyridyldithio)-propionate (SPDP) [92], adipic acid dihydrazide ADH [93], C₄ to C₁₂ moieties [94], *etc.* Carbodiimide condensation can also be used [95].

As described in reference 96, a mixture can include one conjugate with direct saccharide/protein linkage and another conjugate with linkage via a linker. This arrangement applies particularly when using saccharide conjugates from different meningococcal serogroups e.g. Men-A and Men-C saccharides may be conjugated via a linker, whereas Men-W135 and Men-Y saccharides may be conjugated directly to a carrier protein.

Where a composition includes one or more of Men-A, -C, -W and/or -Y conjugates, in some embodiments it can advantageously include a Hib conjugate as well (see below). Where a composition includes saccharide from more than one meningococcal serogroup, there is an mean saccharide mass per serogroup. If substantially equal masses of each serogroup are used then the mean mass will be the same as each individual mass; where non-equal masses are used then the mean will differ e.g. with a 10:5:5:5 μg amount for a Men-ACWY mixture, the mean mass is 6.25μg per serogroup. If a Hib saccharide is also included then, in some embodiments, its mass will be substantially the same as the mean mass of meningococcal saccharide per serogroup. In some embodiments, the mass of Hib saccharide will be more than (e.g. at least 1.5x) the mean mass of meningococcal saccharide per serogroup. In some embodiments, the mass of Hib saccharide will be less than (e.g. at least 1.5x) the mean mass of meningococcal saccharide per serogroup [97].

### The oil-in-water emulsion adjuvant

Various oil-in-water emulsion adjuvants are known, and they typically include at least one oil and at least one surfactant, with the oil(s) and surfactant(s) being biodegradable (metabolisable) and biocompatible. The oil droplets in the emulsion are generally less than 5μm in diameter, and may even have a sub-micron diameter, with these small sizes being achieved with a microfluidiser to provide stable emulsions. Droplets with a size less than 220nm are preferred as they can be subjected to filter sterilization.

The invention can be used with oils such as those from an animal (such as fish) or vegetable source. Sources for vegetable oils include nuts, seeds and grains. Peanut oil, soybean oil, coconut oil, and olive oil, the most commonly available, exemplify the nut oils. Jojoba oil can be used e.g. obtained from the jojoba bean. Seed oils include safflower oil, cottonseed oil, sunflower seed oil, sesame seed oil and the like. In the grain group, corn oil is the most readily available, but the oil of other cereal grains such as wheat, oats, rye, rice, teff, triticale and the like may also be used. 6-10 carbon fatty acid esters of glycerol and 1,2-propanediol, while not occurring naturally in seed oils, may be prepared by hydrolysis, separation and esterification of the appropriate materials starting from the nut and seed oils. Fats and oils from mammalian milk are metabolizable and may therefore be used in the practice of this invention. The procedures for separation, purification, saponification and other means necessary for obtaining pure oils from animal sources are well known in the art. Most fish contain metabolizable oils which may be readily recovered. For example, cod liver oil, shark liver oils, and whale oil such as spermaceti exemplify several of the fish oils which may be used herein. A number of branched chain oils are synthesized biochemically in 5-carbon isoprene units and are generally referred to as terpenoids. Shark liver oil contains a branched, unsaturated terpenoids known as squalene, 2,6,10,15,19,23-hexamethyl-2,6,10,14,18,22-tetracosahexaene, which is particularly preferred herein. Squalane, the saturated analog to squalene, is also a preferred oil. Fish oils, including squalene and squalane, are readily available from commercial sources or may be obtained by methods known in the art. Other preferred oils are the tocopherols. Mixtures of oils can be used.

Where a composition includes a tocopherol, any of the α, β, y, δ, ε or ξ tocopherols can be used, but α-tocopherols are preferred. The tocopherol can take several forms *e.g.* different salts and/or isomers. Salts include organic salts, such as succinate, acetate, nicotinate, *etc.* D-α-tocopherol and DL-α-tocopherol can both be used. A preferred α-tocopherol is DL-α-tocopherol, and the preferred salt of this tocopherol is the succinate.

Surfactants can be classified by their 'HLB' (hydrophile/lipophile balance). Preferred surfactants of the invention have a HLB of at least 10, preferably at least 15, and more preferably at least 16. The invention can be used with surfactants including, but not limited to: the polyoxyethylene sorbitan esters surfactants (commonly referred to as the Tweens), especially polysorbate 20 and polysorbate 80; copolymers of ethylene oxide (EO), propylene oxide (PO), and/or butylene oxide (BO), sold under the DOWFAX™ tradename, such as linear EO/PO block copolymers; octoxynols, which can vary in the number of repeating ethoxy (oxy-1,2-ethanediyl) groups, with octoxynol-9 (Triton X-100, or t-octylphenoxypolyethoxyethanol) being of particular interest; (octylphenoxy)polyethoxyethanol (IGEPAL CA-630/NP-40); phospholipids such as phosphatidylcholine (lecithin); polyoxyethylene fatty ethers derived from lauryl, cetyl, stearyl and oleyl alcohols (known as Brij surfactants), such as triethyleneglycol monolauryl ether (Brij 30); and sorbitan esters (commonly known as the SPANs), such as sorbitan trioleate (Span 85) and sorbitan monolaurate. Preferred surfactants for including in the emulsion are Tween 80 (polyoxyethylene sorbitan monooleate), Span 85 (sorbitan trioleate), lecithin and Triton X-100.

Mixtures of surfactants can be used e.g. Tween 80/Span 85 mixtures. A combination of a polyoxyethylene sorbitan ester such as polyoxyethylene sorbitan monooleate (Tween 80) and an octoxynol such as t-octylphenoxypolyethoxyethanol (Triton X-100) is also suitable. Another useful combination comprises laureth-9 plus a polyoxyethylene sorbitan ester and/or an octoxynol.

Preferred amounts of surfactants (% by weight) are: polyoxyethylene sorbitan esters (such as Tween 80) 0.01 to 1%, in particular about 0.1 %; octyl- or nonylphenoxy polyoxyethanols (such as Triton X-100, or other detergents in the Triton series) 0.001 to 0.1 %, in particular 0.005 to 0.02%; polyoxyethylene ethers (such as laureth 9) 0.1 to 20 %, preferably 0.1 to 10 % and in particular 0.1 to 1 % or about 0.5%.

Specific oil-in-water emulsion adjuvants useful with the invention include, but are not limited to:
● A sub-micron emulsion of squalene, Tween 80, and Span 85. The composition of the emulsion by volume can be about 5% squalene, about 0.5% polysorbate 80 and about 0.5% Span 85. In weight terms, these ratios become 4.3% squalene, 0.5% polysorbate 80 and 0.48% Span 85. This adjuvant is known as 'MF59' [98-100], as described in more detail in Chapter 10 of ref. 101 and chapter 12 of ref. 102. The MF59 emulsion may include citrate ions e.g. 10mM sodium citrate buffer.
● An emulsion of squalene, a tocopherol, and Tween 80. The emulsion may include phosphate buffered saline. It may also include Span 85 (e.g. at 1%) and/or lecithin. These emulsions may have from 2 to 10% squalene, from 2 to 10% tocopherol and from 0.3 to 3% Tween 80, and the weight ratio of squaleneaocopherol is preferably ≤1 as this provides a more stable emulsion. Squalene and Tween 80 may be present volume ratio of about 5:2. One such emulsion can be made by dissolving Tween 80 in PBS to give a 2% solution, then mixing 90ml of this solution with a mixture of (5g of DL-α-tocopherol and 5ml squalene), then microfluidising the mixture. The resulting emulsion may have sub-micron oil droplets e.g. with an average diameter of between 100 and 250nm, preferably about 180nm.
● An emulsion of squalene, a tocopherol, and a Triton detergent (*e.g*. Triton X-100). The emulsion may also include a 3d-MPL. The emulsion may contain a phosphate buffer.
● An emulsion comprising a polysorbate (*e.g*. polysorbate 80), a Triton detergent (*e.g*. Triton X-100) and a tocopherol (*e.g*. an α-tocopherol succinate). The emulsion may include these three components at a mass ratio of about 75:11:10 (*e.g*. 750μg/ml polysorbate 80, 110μg/ml Triton X-100 and 100μg/ml α-tocopherol succinate), and these concentrations should include any contribution of these components from antigens. The emulsion may also include squalene. The emulsion may also include a 3d-MPL. The aqueous phase may contain a phosphate buffer.
● An emulsion of squalane, polysorbate 80 and poloxamer 401 ("Pluronic™ L121"). The emulsion can be formulated in phosphate buffered saline, pH 7.4. This emulsion is a useful delivery vehicle for muramyl dipeptides, and has been used with threonyl-MDP in the "SAF-1" adjuvant [103] (0.05-1% Thr-MDP, 5% squalane, 2.5% Pluronic L121 and 0.2% polysorbate 80). It can also be used without the Thr-MDP, as in the "AF" adjuvant [104] (5% squalane, 1.25% Pluronic L121 and 0.2% polysorbate 80). Microfluidisation is preferred.
● An emulsion comprising squalene, an aqueous solvent, a polyoxyethylene alkyl ether hydrophilic nonionic surfactant (*e.g.* polyoxyethylene (12) cetostearyl ether) and a hydrophobic nonionic surfactant (*e.g*. a sorbitan ester or mannide ester, such as sorbitan monoleate or 'Span 80'). The emulsion is preferably thermoreversible and/or has at least 90% of the oil droplets (by volume) with a size less than 200 nm [105]. The emulsion may also include one or more of: alditol; a cryoprotective agent (*e.g*. a sugar, such as dodecylmaltoside and/or sucrose); and/or an alkylpolyglycoside. Such emulsions may be lyophilized.
● An emulsion having from 0.5-50% of an oil, 0.1-10% of a phospholipid, and 0.05-5% of a non-ionic surfactant. As described in reference 106, preferred phospholipid components are phosphatidylcholine, phosphatidylethanolamine, phosphatidylserine, phosphatidylinositol, phosphatidylglycerol, phosphatidic acid, sphingomyelin and cardiolipin. Sub-micron droplet sizes are advantageous.
● A sub-micron oil-in-water emulsion of a non-metabolisable oil (such as light mineral oil) and at least one surfactant (such as lecithin, Tween 80 or Span 80). Additives may be included, such as QuilA saponin, cholesterol, a saponin-lipophile conjugate (such as GPI-0100, described in reference 107, produced by addition of aliphatic amine to desacylsaponin via the carboxyl group of glucuronic acid), dimethyidioctadecylammonium bromide and/or N,N-dioctadecyl-N,N-bis (2-hydroxyethyl)propanediamine.
● An emulsion comprising a mineral oil, a non-ionic lipophilic ethoxylated fatty alcohol, and a non-ionic hydrophilic surfactant (*e.g*. an ethoxylated fatty alcohol and/or polyoxyethylene-polyoxypropylene block copolymer) [108].
● An emulsion comprising a mineral oil, a non-ionic hydrophilic ethoxylated fatty alcohol, and a non-ionic lipophilic surfactant (e.g. an ethoxylated fatty alcohol and/or polyoxyethylene-polyoxypropylene block copolymer) [108].
● An emulsion in which a saponin (e.g. QuilA or QS21) and a sterol *(e.g.* a cholesterol) are associated as helical micelles [109].

Oil-in-water emulsions can be used as adjuvants on their own, or as carriers for further immunostimulatory compounds e.g. immunostimulatory oligonucleotides, 3d-MPL, *etc.*

3dMPL (also known as 3 de-O-acylated monophosphoryl lipid A or 3-O-desacyl-4'-monophosphoryl lipid A) is an adjuvant in which position 3 of the reducing end glucosamine in monophosphoryl lipid A has been de-acylated. 3dMPL has been prepared from a heptoseless mutant of *Salmonella minnesota,* and is chemically similar to lipid A but lacks an acid-labile phosphoryl group and a base-labile acyl group. Preparation of 3dMPL was originally described in reference 110.

### Reconstitution and packaging

Lyophilised antigen components of the invention will ultimately be reconstituted with a liquid component to give material suitable for administration to a patient. The reconstitution will typically take place at the point of use. Thus an antigen and an oil-in-water emulsion adjuvant may be kept separately in a packaged or distributed vaccine kit, ready for final formulation at the time of use.

In a kit containing two containers, one will include liquid for reconstitution and the second container includes lyophilised material. The second container will usually be hermetically sealed. The liquid will usually be introduced into the second container via a first needle, thereby reconstituting the lyophilised material into a liquid form. The liquid will then be withdrawn, usually into a syringe, for administration to a patient. This withdrawal step may be via the first needle, but will often be via a second needle. The needle used for the withdrawal may be the same needle that is then used for patient injection, or it may be different.

The second container will typically be a vial. An oil-in-water emulsion for reconstituting the lyophilised material may also located in a vial but, as an alternative, may be located in a syringe. A further arrangement has the first and second containers as separate chambers in a dual-chamber syringe such that, when actuated, liquid material is introduced from the first container into the second container. The mixed and reconstituted materials can then exit the syringe in liquid form. In all cases, however, the lyophilised and liquid materials are kept separate until ready for mixing.

Although an oil-in-water emulsion will usually be used in its liquid form, in some embodiments of the invention it is possible to use a lyophilised oil-in-water emulsion adjuvant. Lyophilisation of emulsion adjuvants in this way is disclosed in, for instance, references 105 and 111. These dried emulsions will still be reconstituted into liquid form at the time of use e.g. using an aqueous carrier. The lyophilised adjuvant and lyophilised antigen components may be separate kit components, but in some embodiments they may be mixed (either pre- or post-lyophilisation) in lyophilised form. Thus, in some embodiments, the invention provides a composition comprising a mixture of a lyophilised oil-in-water emulsion and a lyophilised antigenic composition comprising an immunogen for raising an immune response against *Neisseria meningitidis* serogroup B. This mixed lyophilised composition can be mixed with an aqueous carrier to give in one reconstitution step a Men-B composition with an oil-in-water emulsion adjuvant.

Thus a kit may comprise, for instance, two vials, one ready-filled syringe and one vial, *etc.* A syringe will generally include a single dose of the composition, whereas a vial may include a single dose or multiple doses. For multiple dose forms, therefore, vials are preferred to pre-filled syringes.

Further liquid and/or lyophilised materials may also be added prior to administration to a patient.

A container for a lyophilised component may have a cap (e.g. a Luer lock) adapted such that a pre-filled syringe can be inserted into the cap, the contents of the syringe can be expelled into the vial to reconstitute the freeze-dried material therein, and the contents of the vial can be removed back into the syringe. After removal of the syringe from the vial, a needle can then be attached and the vaccine can be administered to a patient. The cap may be located inside a seal or cover, such that the seal or cover has to be removed before the cap can be accessed.

Where a component is packaged into a vial, this are preferably made of a glass or plastic material. The vial is preferably sterilized before material is added to it. To avoid problems with latex-sensitive patients, vials can be sealed with a latex-free stopper. The vial may include a single dose of vaccine, or it may include more than one dose (a 'multidose' vial) e.g. 10 doses. Preferred vials are made of colourless glass.

Where the vaccine is packaged into a syringe, the syringe may have a needle attached to it, or it may be needle-free. A separate needle may be supplied with the syringe for assembly and use. Safety needles are preferred. 1-inch 23-gauge, 1-inch 25-gauge and 5/8-inch 25-gauge needles are typical. Syringes may be provided with peel-off labels on which the lot number and expiration date of the contents may be printed, to facilitate record keeping. The plunger in the syringe preferably has a stopper to prevent the plunger from being accidentally removed during aspiration.

Where a glass container (e.g. a syringe or a vial) is used, then it is preferred to use a container made from a borosilicate glass rather than from a soda lime glass.

As vaccines are usually administered to patients in 0.5ml doses, the volume of the liquid in the first container will be suitable for giving a dosage volume after reconstitution of at least 0.5ml e.g. 0.6ml, accounting for wastage volume.

For stability reasons, the lyophilised component of the invention may include a stabiliser such as lactose, sucrose and/or mannitol, as well as mixtures thereof e.g. lactose/sucrose mixtures, sucrose/mannitol mixtures, *etc.* Using a sucrose/mannitol mixture can speed up the drying process. A lyophilised component may also include sodium chloride. Soluble components in the lyophilised material will be retained in the composition after reconstitution, and so final liquid vaccines may thus contain lactose and/or sucrose.

A composition may include a temperature protective agent, as described in reference 112. Examples include glycerin, propylene glycol, and/or polyethylene glycol (PEG). Suitable PEGs may have an average molecular weight ranging from 200-20,000 Da. In a preferred embodiment, the polyethylene glycol can have an average molecular weight of about 300 Da ('PEG-300').

### Pharmaceutical compositions

Materials of the invention will ultimately be used to prepare pharmaceutical compositions for administration to a patient. These will typically include a pharmaceutically acceptable carrier. A thorough discussion of pharmaceutically acceptable carriers is available in reference 113.

Effective dosage volumes can be routinely established, but a typical human dose of the composition has a volume of about 0.5ml e.g. for intramuscular injection. The RIVM OMV-based vaccine was administered in a 0.5ml volume [114] by intramuscular injection to the thigh or upper arm. MeNZB™ is administered in a 0.5ml by intramuscular injection to the anterolateral thigh or the deltoid region of the arm. Similar doses may be used for other delivery routes e.g. an intranasal OMV-based vaccine for atomisation may have a volume of about 100μl or about 130μl per spray, with four sprays administered to give a total dose of about 0.5ml.

The pH of a composition after reconstitution is preferably between 6 and 8, and more preferably between 6.5 and 7.5 (e.g. about 7). The pH of the RIVM OMV-based vaccine is 7.4 [115], and a pH <7.5 is preferred for compositions of the invention. The RIVM OMV-based vaccine maintains pH by using a 10mM Tris/HCl buffer, and stable pH in compositions of the invention may be maintained by the use of a buffer e.g. a Tris buffer, a citrate buffer, phosphate buffer, or a histidine buffer. Thus compositions of the invention will generally include a buffer. The buffer components will be located in the liquid component and/or the lyophilised component, as appropriate, to give the final post-reconstitution arrangement as desired.

The composition may be sterile and/or pyrogen-free. Compositions of the invention may be isotonic with respect to humans.

Compositions of the invention for administration to patients are immunogenic, and are more preferably vaccine compositions. Vaccines according to the invention may either be prophylactic (i. e. to prevent infection) or therapeutic (i.e. to treat infection), but will typically be prophylactic. Immunogenic compositions used as vaccines comprise an immunologically effective amount of antigen(s), as well as any other components, as needed. By 'immunologically effective amount', it is meant that the administration of that amount to an individual, either in a single dose or as part of a series, is effective for treatment or prevention. This amount varies depending upon the health and physical condition of the individual to be treated, age, the taxonomic group of individual to be treated (e.g. non-human primate, primate, *etc.),* the capacity of the individual's immune system to synthesise antibodies, the degree of protection desired, the formulation of the vaccine, the treating doctor's assessment of the medical situation, and other relevant factors. It is expected that the amount will fall in a relatively broad range that can be determined through routine trials. The antigen content of compositions of the invention will generally be expressed in terms of the amount of protein per dose. A dose of about 0.9 mg protein per ml is typical for OMV-based intranasal vaccines.

Meningococci affect various areas of the body and so the compositions of the invention may be prepared in various liquid forms. For example, the compositions may be prepared as injectables, either as solutions or suspensions. The composition may be prepared for pulmonary administration e.g. by an inhaler, using a fine spray. The composition may be prepared for nasal, aural or ocular administration e.g. as spray or drops. Injectables for intramuscular administration are typical.

Compositions of the invention may include an antimicrobial, particularly when packaged in multiple dose format. Antimicrobials such as thiomersal and 2-phenoxyethanol are commonly found in vaccines, but it is preferred to use either a mercury-free preservative or no preservative at all.

A lyophilised component of the invention and/or a co-packaged oil-in-water emulsion adjuvant may be substantially free from aluminium salts. This arrangement permits a reconstituted composition of the invention to be substantially free from aluminium salts.

Compositions of the invention may comprise detergent e.g. a Tween (polysorbate), such as Tween 80. Detergents are generally present at low levels e.g. <0.01%.

Compositions of the invention may include residual detergent (e.g. deoxycholate) from OMV preparation. The amount of residual detergent is preferably less than 0.4μg (more preferably less than 0.2μg) for every μg of Men-B protein.

Compositions of the invention may include LOS from meningococcus. The amount of LOS is preferably less than 0.12μg (more preferably less than 0.05μg) for every μg of protein.

Compositions of the invention may include sodium salts (e.g. sodium chloride) to give tonicity. A concentration of 10±2 mg/ml NaCl is typical e.g. about 9 mg/ml.

### Methods of treatment

The invention also provides a method for raising an immune response in a mammal, comprising administering a liquid pharmaceutical composition of the invention to the mammal. The immune response is preferably protective and preferably involves antibodies. The method may raise a booster response in a patient that has already been primed against *N.meningitidis.* Subcutaneous and intranasal prime/boost regimes for OMVs are disclosed in ref. 116.

The mammal is preferably a human. Where the vaccine is for prophylactic use, the human is preferably a child (e.g. a toddler or infant) or a teenager; where the vaccine is for therapeutic use, the human is preferably an adult. A vaccine intended for children may also be administered to adults e.g. to assess safety, dosage, immunogenicity, *etc.*

The invention also provides compositions and mixtures of the invention for use as a medicament. The medicament is preferably able to raise an immune response in a mammal (i.e. it is an immunogenic composition) and is more preferably a vaccine.

The invention also provides the use of compositions and mixtures of the invention in the manufacture of a medicament for raising an immune response in a mammal. The invention also provides the use of (i) an adjuvant comprising an oil-in-water emulsion; and (ii) a lyophilised antigenic composition comprising an immunogen for raising an immune response against *N.meningitidis* serogroup B, in the manufacture of a medicament for raising an immune response in a mammal. The use may also involve (iii) a conjugated capsular saccharide from one or more of *N.meningitidis* serogroups A, C, W135 and/or Y.

These uses and methods are preferably for the prevention and/or treatment of a disease caused by *N.meningitidis e.g.* bacterial (or, more specifically, meningococcal) meningitis, or septicemia.

One way of checking efficacy of therapeutic treatment involves monitoring Neisserial infection after administration of the composition of the invention. One way of checking efficacy of prophylactic treatment involves monitoring immune responses against antigens after administration of the composition. Immunogenicity of compositions of the invention can be determined by administering them to test subjects (e.g. children 12-16 months age, or animal models [117]) and then determining standard parameters including serum bactericidal antibodies (SBA) and ELISA titres (GMT). These immune responses will generally be determined around 4 weeks after administration of the composition, and compared to values determined before administration of the composition. A SBA increase of at least 4-fold or 8-fold is preferred. Where more than one dose of the composition is administered, more than one post-administration determination may be made.

In general, compositions of the invention are able to induce serum bactericidal antibody responses after being administered to a subject. These responses are conveniently measured in mice and are a standard indicator of vaccine efficacy. Serum bactericidal activity (SBA) measures bacterial killing mediated by complement, and can be assayed using human or baby rabbit complement. WHO standards require a vaccine to induce at least a 4-fold rise in SBA in more than 90% of recipients. MeNZB™ elicits a 4-fold rise in SBA 4-6 weeks after administration of the third dose.

Preferred compositions can confer an antibody titre in a human subject patient that is superior to the criterion for seroprotection for an acceptable percentage of subjects. Antigens with an associated antibody titre above which a host is considered to be seroconverted against the antigen are well known, and such titres are published by organisations such as WHO. Preferably more than 80% of a statistically significant sample of subjects is seroconverted, more preferably more than 90%, still more preferably more than 93% and most preferably 96-100%.

Compositions of the invention will generally be administered directly to a patient. Direct delivery may be accomplished by parenteral injection (e.g. subcutaneously, intraperitoneally, intravenously, intramuscularly, or to the interstitial space of a tissue), or by any other suitable route. The invention may be used to elicit systemic and/or mucosal immunity. Intramuscular administration to the thigh or the upper arm is preferred. Injection may be via a needle (e.g. a hypodermic needle), but needle-free injection may alternatively be used. A typical intramuscular dose is 0.5 ml.

Dosage treatment can be a single dose schedule or a multiple dose schedule. Multiple doses may be used in a primary immunisation schedule and/or in a booster immunisation schedule. A primary dose schedule may be followed by a booster dose schedule. Suitable timing between priming doses (e.g. between 4-16 weeks), and between priming and boosting, can be routinely determined. The OMV-based RIVM vaccine was tested using a 3- or 4-dose primary schedule, with vaccination at 0. 2 & 8 or 0, 1, 2 & 8 months. MeNZB™ is administered as three doses at six week intervals.

Compositions of the invention may be used to induce bactericidal antibody responses against more than one hypervirulent lineage of meningococcus. In particular, they can preferably induce bactericidal responses against two or three of the following three hypervirulent lineages: (i) cluster A4; (ii) ET5 complex; and (iii) lineage 3. They may additionally induce bactericidal antibody responses against one or more of hypervirulent lineages subgroup I, subgroup III, subgroup IV-1 or ET-37 complex, and against other lineages e.g. hyperinvasive lineages. This does not necessarily mean that the composition can induce bactericidal antibodies against each and every strain of meningococcus within these hypervirulent lineages e.g. rather, for any given group of four of more strains of meningococcus within a particular hypervirulent lineage, the antibodies induced by the composition are bactericidal against at least 50% (e.g. 60%, 70%, 80%, 90% or more) of the group. Preferred groups of strains will include strains isolated in at least four of the following countries: GB, AU, CA, NO, IT, US, NZ, NL, BR, and CU. The serum preferably has a bactericidal titre of at least 1024 (*e.g*. 2¹⁰, 2¹¹, 2¹², 2¹³, 2¹⁴, 2¹⁵, 2¹⁶, 2¹⁷, 2¹⁸, or higher, preferably at least 2¹⁴) *e.g*. the serum is able to kill at least 50% of test bacteria of a particular strain when diluted 1/1024.

Useful compositions can induce bactericidal responses against the following strains of serogroup B meningococcus: (i) from cluster A4, strain 961-5945 (B:2b:P1.21,16) and/or strain G2136 (B:-); (ii) from ET-5 complex, strain MC58 (B:15:P1.7,16b) and/or strain 44/76 (B:15:P1.7,16); (iii) from lineage 3, strain 394/98 (B:4:P1.4) and/or strain BZ198 (B:NT:-). More preferred compositions can induce bactericidal responses against strains 961-5945, 44/76 and 394/98.

Strains 961-5945 and G2136 are both *Neisseria* MLST reference strains [ids 638 & 1002 in ref. 118]. Strain MC58 is widely available (e.g. ATCC BAA-335) and was the strain sequenced in reference 119. Strain 44/76 has been widely used and characterised (e.g. ref. 120) and is one of the *Neisseria* MLST reference strains [id 237 in ref. 118; row 32 of Table 2 in ref. 19]. Strain 394/98 was originally isolated in New Zealand in 1998, and there have been several published studies using this strain (e.g. refs. 121 & 122). Strain BZ198 is another MLST reference strain (id 409 in ref. 118; row 41 of Table 2 in ref. 19).

### Further compositions of the invention

The invention provides a lyophilised antigenic composition comprising an immunogen for raising an immune response against *N.meningitidis* serogroup B, wherein the immunogen comprises Men-B outer membrane vesicles as described above, provided that the composition does not include vesicles from any of strains: F91; JB10124; or HP10124.

The invention provides a lyophilised antigenic composition comprising an immunogen for raising an immune response against *N.meningitidis* serogroup B, wherein the immunogen comprises Men-B outer membrane vesicles as described above, wherein the vesicles are from a strain with a L2 or a L3 immunotype. The composition may include vesicles from both a L2 and a L3 strain.

The invention provides a lyophilised antigenic composition comprising an immunogen for raising an immune response against *N.meningitidis* serogroup B, wherein the immunogen comprises Men-B outer membrane vesicles as described above, wherein the vesicles include LOS that does not include a sialyl-lacto-N-neotetraose epitope.

The invention provides a lyophilised antigenic composition comprising an immunogen for raising an immune response against *N.meningitidis* serogroup B, wherein the immunogen comprises a purified fHBP protein. The composition may include more than one variant of fHBP, as described above.

These lyophilised compositions are suitable for reconstitution by an adjuvant comprising an oil-in-water emulsion, and they are thus suitable for use as kit components of the invention or for use in the methods of the invention, *etc.* As they may be sold or distributed without emulsion adjuvants, though, they are independent embodiments of the invention. They may, however, be packaged in kit form in combination with another container comprising a liquid adjuvant. This liquid adjuvant preferably comprises an oil-in-water emulsion.

The invention also provides an adjuvanted antigenic composition comprising *N.meningitidis* serogroup B membrane vesicles and a sub-micron oil-in-water emulsion. The emulsion preferably comprises squalene and/or polysorbate 80. The emulsion's oil droplets are ideally <500nm diameter. The vesicles may over-express one or more proteins as discussed above, and/or may include one or more of knockout mutations as discussed above e.g. down-regulated or knocked-out LgtB and/or GalE to truncate LOS, up-regulated TbpA, *etc.* "Intra-bleb" conjugation may be used. This adjuvanted composition may be prepared by mixing lyophilised antigens with an emulsion, as described above, or in contrast may be prepared by using an aqueous vesicle preparation.

### Further antigenic components

As well as containing antigens from *N.meningitidis,* compositions may include antigens from further pathogens. For example, the composition may comprise one or more of the following further antigens:
- an antigen from *Streptococcus pneumoniae,* such as a saccharide (typically conjugated)
- an antigen from hepatitis B virus, such as the surface antigen HBsAg.
- an antigen from *Bordetella pertussis,* such as pertussis holotoxin (PT) and filamentous haemagglutinin (FHA) from *B.pertussis,* optionally also in combination with pertactin and/or agglutinogens 2 and 3.
- a diphtheria antigen, such as a diphtheria toxoid.
- a tetanus antigen, such as a tetanus toxoid.
- a saccharide antigen from *Haemophilus influenzae* B (Hib), typically conjugated.
- inactivated poliovirus antigens.

These additional antigens may be included in liquid form in the same container as the oil-in-water emulsion, in lyophilised form in the same container as the lyophilised Men-B antigen, or in a third container (either in lyophilised or, usually, in liquid form).

Where a diphtheria antigen is included in the composition it is preferred also to include tetanus antigen and pertussis antigens. Similarly, where a tetanus antigen is included it is preferred also to include diphtheria and pertussis antigens. Similarly, where a pertussis antigen is included it is preferred also to include diphtheria and tetanus antigens. DTP combinations are thus preferred.

If a Hib saccharide is included (typically as a conjugate), the saccharide moiety may be a polysaccharide (e.g. full-length polyribosylribitol phosphate (PRP) as purified from bacteria), but it is also possible to fragment the purified saccharide to make oligosaccharides (e.g. MW from ∼1 to ∼5 kDa) e.g. by hydrolysis. The concentration of Hib conjugate in a reconstituted vaccine will usually be in the range of 0.5μg to 50μg e.g. from 1-20μg, from 10-15μg, from 12-16μg, etc. The amount may be about 15g, or about 12.5μg in some embodiments. A mass of less than 5μg may be suitable [123] e.g. in the range 1-5μg, 2-4μg, or about 2.5μg. As described above, in combinations that include Hib saccharide and meningococcal saccharides, the dose of the former may be selected based on the dose of the latter (in particular, with multiple meningococcal serogroups, their mean mass). Further characteristics of Hib conjugates are as disclosed above for meningococcal conjugates, including choice of carrier protein *(e.g.* CRM197 or tetanus toxoid), linkages, ratios, *etc.*

If a *S.pneumoniae* antigen is included, this may be a polypeptide or a saccharide. Conjugates capsular saccharides are particularly useful for immunising against pneumococcus. The saccharide may be a polysaccharide having the size that arises during purification of the saccharide from bacteria, or it may be an oligosaccharide achieved by fragmentation of such a polysaccharide. In the 7-valent PREVNAR™ product, for instance, 6 of the saccharides are presented as intact polysaccharides while one (the 18C serotype) is presented as an oligosaccharide. A composition may include a capsular saccharide from one or more of the following pneumococcal serotypes: 1, 2, 3, 4, 5, 6A, 6B, 7F, 8, 9N, 9V, 10A, 11A, 12F, 14, 15B, 17F, 18C, 19A, 19F, 20, 22F, 23F and/or 33F. A composition may include multiple serotypes e.g. 2, 3, 4, 5, 6, 7, 8, 9, 10, 11, 12, 13, 14, 15, 16, 17, 18, 19, 20, 21, 22, 23 or more serotypes. 7-valent, 9-valent, 10-valent, 11-valent and 13-valent conjugate combinations are already known in the art, as is a 23-valent unconjugated combination. For example, an 10-valent combination may include saccharide from serotypes 1 , 4, 5, 6B, 7F, 9V, 14, 18C, 19F and 23F. An 11-valent combination may further include saccharide from serotype 3. A 12-valent combination may add to the 10-valent mixture: serotypes 6A and 19A; 6A and 22F; 19A and 22F; 6A and 15B; 19A and 15B; r 22F and 15B; A 13-valent combination may add to the 11-valent mixture: serotypes 19A and 22F; 8 and 12F; 8 and 15B; 8 and 19A; 8 and 22F; 12F and 15B; 12F and 19A; 12F and 22F; 15B and 19A; 15B and 22F. *etc.* Further characteristics of pneumococcal conjugates are as disclosed above for meningococcal conjugates, including choice of carrier protein (e.g. CRM197 or tetanus toxoid), linkages, ratios, *etc.* Where a composition includes more than one conjugate, each conjugate may use the same carrier protein or a different carrier protein. Reference 124 describes potential advantages when using different carrier proteins in multivalent pneumococcal conjugate vaccines.

### General

The term "comprising" encompasses "including" as well as "consisting" e.g. a composition "comprising" X may consist exclusively of X or may include something additional e.g. X + Y.

The term "about" in relation to a numerical value x means, for example, *x*±10%.

The word "substantially" does not exclude "completely" e.g. a composition which is "substantially free" from Y may be completely free from Y. Where necessary, the word "substantially" may be omitted from the definition of the invention.

### BRIEF DESCRIPTION OF THE DRAWINGS

Figure 1 shows two superimposed analytical traces for a composition of the invention. The lines are a pre-lyophilisation and a post-reconstitution composition. Essentially only one line is visible because they are so closely similar
Figure 2 shows two superimposed analytical traces for a composition stored at 4°C and a composition stored at 37°C.Unlike Figure 1, two lines are visible.
Figure 3 shows SDS-PAGE analysis of various formulations, The 10 lanes, from left to right, show: (1) MW marker; (2)-(4) liquid antigens at 100μg/ml, 50μg/ml and 25μg/ml; (5) antigens in mixture of 2% mannitol and 3% sucrose, prior to lyophilisation; (6) as lane (5), but after lyophilisation and reconstitution with wfi; (7) as lane (5), but after lyophilisation and reconstitution with MF59; (8) to (10) as lanes (5) to (7), but in 5% sucrose.

### MODES FOR CARRYING OUT THE INVENTION

### Inclusion of adjuvant in Men-B vaccine

Initial pre-clinical assessment of the Novartis Men-B vaccine indicated that an optimum immune response required the presence of aluminium hydroxide adjuvant. Even in the presence of this adjuvant, however, strain coverage was incomplete. For example, whereas 100% of tested ST32 and ST8 strains were killed by sera elicited by the vaccine, this figure dropped to 65% for ST11 strains. In contrast, the use of the MF59 emulsion as the adjuvant provided 100% coverage for all of ST32, ST8 and ST11 strains. Further experiments confirmed MF59's superiority.

The same superiority was seen when conjugated capsular saccharides from serogroups A, C, W135 and Y were added to the Men-B vaccine. The immunogenicity achieved by this A-B-C-W-Y vaccine was better using MF59 than when using aluminium hydroxide, both in terms of bactericidal titres and strain coverage.

MF59 thus provides an enhanced immunogenic efficacy when compared to the aluminium hydroxide adjuvant. When the stability of this was tested, however, it was found that the Men-B antigens were starting to degrade after around 12 weeks even when stored at 4°C. When stored at higher temperatures then degradation was evident after as early as 2 weeks, with complete degradation after 6 months. Analysis using the Agilent 2100 Bioanalyzer or size exclusion chromatography confirmed the degradation. In contrast, the antigens remained stable when adsorbed to aluminium hydroxide.

Reduced stability was also seen for conjugated capsular saccharides from non-B serogroups in MF59. The level of free sialic acid (a component in the capsular saccharides of Men-C, Men-W135 and Men-Y) rose gradually in a MF59-adjuvanted formulation stored at 4°C, reaching about 15% after 6 months. At higher temperatures, however, the free level reached 50% after about 10 weeks and 100% (i.e. total degradation) in 6 months.

Thus the enhanced immunogenicity achieved by MF59 is at the expense of storage stability. Work was performed to see if a stable formulation could be achieved while using MF59 and/or without requiring adsorption to an aluminium hydroxide adjuvant.

### Men-B lyophilisation

In an attempt to achieve the stability goal, the Men-B antigens were lyophilised. After reconstitution, it was confirmed that their efficacy was retained. Moreover, stability was seen for mixtures of Men-B antigens with conjugated capsular saccharides from each of serogroups A, C, W135 and Y.

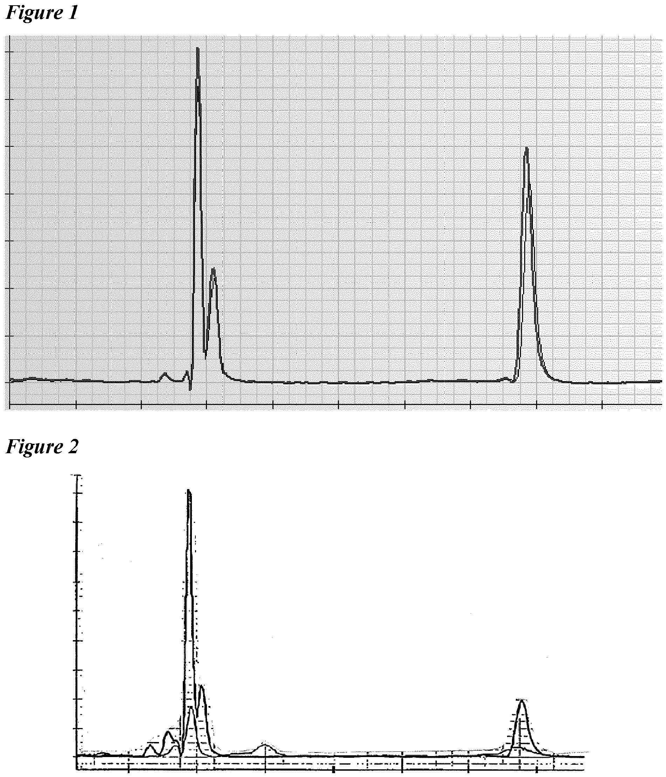

For instance, Figure 1 shows two superimposed analytical traces, with the peaks corresponding to the elution positions of Men-B proteins. The traces are almost identical, revealing no substantial physicochemical changes. In contrast, Figure 2 shows two superimposed traces of the same composition stored at either 4°C or 37°C, and the changes are readily visible. Other analytical techniques confirmed the absence of any detectable changes pre- and post-lyophilisation. The integrity of the individual Men-B antigens appeared to be conserved even after 6 months of post-lyophilisation storage at 4°C.

The compositions had been lyophilised in the presence of 4.5% mannitol and 1.5% sucrose.

Thus long-term stability of the meningococcal antigens can be achieved without needing adsorption to an aluminium salt. Thus lyophilisation permits the antigens to be used in combination with an oil-in-water emulsion, thereby providing the enhanced efficacy and strain coverage that have been demonstrated for these adjuvants while avoiding the associated stability problems.

### Further formulations

In further development work for a lyophilised presentation of the Men-B vaccine, two formulations of the recombinant protein vaccine were prepared. Both used sucrose as a lyophilisation stabiliser, but one additionally included mannitol. Osmolarity is 300mOsmU and pH is 7.0. Each vial includes enough material for one human dose with a 40% excess (70μg of each recombinant protein, 15mg PBS, and either 14mg mannitol or 21mg mannitol + 35mg sucrose), and will be reconstituted with 700μl water of MF59 (or, for comparison, with wfi).

Moisture levels were measured immediately after lyophilisation and then for a month at low or elevated temperatures. The moisture content remained constant at about 1.1%.

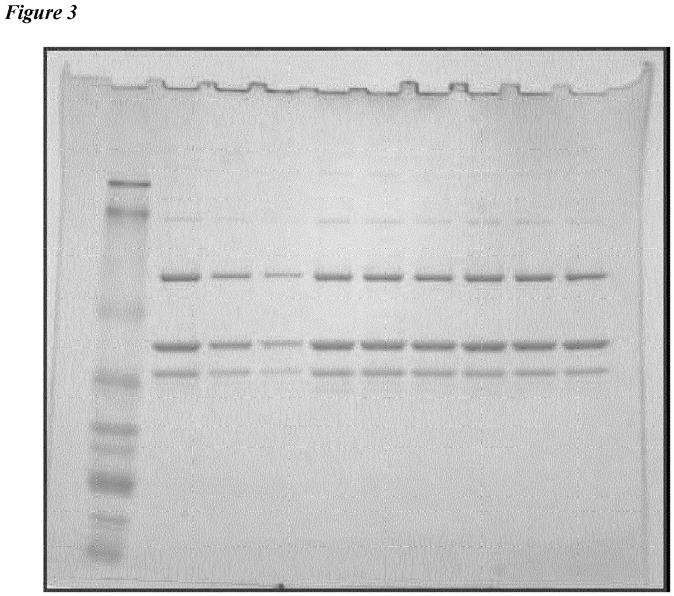

A comparison of RP-HPLC traces before lyophilisation and after reconstitution showed that the proteins in both formulations remain stable after lyophilisation even for 1 month at 37°C. SDS-PAGE (Figure 3) and western blots also showed that the three proteins were stable after the lyophilisation process, with no evidence of degradation or aggregation after reconstitution with either MF59 or wfi at both 4°C and 37°C. Experion analysis gave the same result. Size exclusion chromatography showed that lyophilisation caused a small increase in aggregation, but the amount of aggregate did not increase thereafter, even after 3 months at 37°C or 6 months at 4°C.

### Inmmnogenicity studies

Mice were used in an immunogenicity study to assess the effect of lyophilisation on vaccine potency. Lyophilised formulations were reconstituted with MF59 and titers were compared against the same antigens in liquid form and extemporaneously mixed with MF59 (as in a 'two vial' approach). The formulations induced similar titers.

To study longer-term stability two lyophilised antigen preparations (one lyophilised with sucrose, the other with sucrose+mannitol) were stored at 4°C and their immunogenicities were tested after 3 and 6 months of storage. The stored antigens were reconstituted with MF59 (also stored at 4°C with the antigens) and quickly used for immunisation. For comparison, freshly-prepared aqueous antigens and MF59 were also mixed and tested in parallel.

ELISA results from two separate studies indicated that the lyophilized formulations, when reconstituted with MF59, induced similar antibody titers (GMT) to those elicited by the freshly-prepared formulation. The same was seen when antibody responses were assessed by SBA e.g. a SBA titer of 32768 was seen at time zero with the sucrose-lyophilised antigen and was still seen after 6 months of storage. Thus the Men-B antigens remain active after lyophilisation and storage.

In further studies the lyophilised preparations were stored at either 4°C or 37°C and immunogenicity was then assessed. Even after 1 month of storage at 37°C the lyophilised antigens showed no loss in SBA activity.

### Size stability of emulsion mixed with lyophilised Men-B antigens

The oil droplet size of a MF59 emulsion was measured over a 24 hour period at 4°C and 25°C, either as emulsion alone, or mixed with lyophilised Men-B antigens, or mixed with a control antigen. Droplet sizes (nm) were as follows:

| **Temp (°C):** | **25** | | | | **4** | |
|---|---|---|---|---|---|---|
| **Time (hrs):** | **0** | **3** | **5** | **24** | **0** | **24** |
| **MF59 alone** | 171 | 169 | 172 | 168 | 172 | 170 |
| **MF59 + control Ag** | 173 | 170 | 167 | 172 | 169 | 172 |
| **MF59 + Men-B_{lyo}** | 169 | 168 | 176 | 170 | 173 | 171 |

Thus the particle size of the emulsion in the presence of the lyophilised Men-B antigens is stable for 24 hours at 4°C or 25°C and is essentially the same as the emulsion on its own.

It will be understood that the invention has been described by way of example only and modifications may be made whilst remaining within the scope and spirit of the invention.

### REFERENCES

[1] Giuliani et al. (2006) Proc Natl Acad Sci USA 103(29):10834-9.
[2] Boutriau et al. (2007) Clin Vaccin Immunol 14:65-73.
[3] WO99/61053.
[4] WO00/50075.
[5] WO2006/100109.
[6] Shi & Schofield (2004) Exp Opin Drug Safety 3:153-8.
[7] Belshe et al. (1998) AIDS. 12(18):2407-15.
[8] Arigita et al. (2004) Vaccine 22:629-42.
[9] Granoff et al. (1997) Infect Immun 65:1710-5.
[10] WO02/09643.
[11] Katial et al. (2002) Infect. Immun. 70:702-707.
[12] US patent 6,180,111.
[13] WO01/34642.
[14] WO2004/019977.
[15] European patent 0011243.
[16] Fredriksen et al. (1991) NIPH Ann. 14(2):67-80.
[17] WO01/91788.
[18] WO2005/004908.
[19] Maiden et al. (1998) PNAS USA 95:3140-3145.
[20] WO98/56901.
[21] WO2006/081259.
[22] Claassen *et al.* (1996) 14(10):1001-8.
[23] WO99/10497.
[24] Steeghs et al. (2001) The EMBO Journal 20:6937-6945.
[25] WO00/25811.
[26] WO01/09350.
[27] WO02/09746.
[28] WO02/062378.
[29] WO2004/014417.
[30] WO03/105890.
[31] WO2006/024946
[32] WO99/24578.
[33] WO99/36544.
[34] WO99/57280.
[35] WO00/22430.
[36] WO96/29412.
[37] WO01/64920.
[38] WO03/020756.
[39] WO2004/048404.
[40] WO2004/032958.
[41] WO98/53851
[42] US-6531131
[43] WO00/26384.
[44] US-6645503
[45] WO01/52885.
[46] Costantino et al. (1992) Vaccine 10:691-8.
[47] Lieberman et al. (1996) JAMA 275:1499-503.
[48] WO02/058737.
[49] WO03/007985.
[50] Rennels et al. (2002) Pediatr Infect Dis J 21:978-979.
[51] Campbell et al. (2002) J Infect Dis 186:1848-1851.
[52] WO03/080678.
[53] Glode et al. (1979) J Infect Dis 139:52-56
[54] WO94/05325; US patent 5,425,946.
[55] Arakere & Frasch (1991) Infect. Immun. 59:4349-4356.
[56] Michon et al. (2000) Dev. Biol. 103:151-160.
[57] Rubinstein & Stein (1998) J. Immunol. 141:4357-4362.
[58] W02005/033148
[59] W02007/000314.
[60] Research Disclosure, 453077 (Jan 2002)
[61] EP-A-0372501.
[62] EP-A-0378881.
[63] EP-A-0427347.
[64] WO93/17712
[65] WO94/03208.
[66] WO98/58668.
[67] EP-A-0471177.
[68] WO91/01146
[69] Falugi et al. (2001) Eur J Immunol 31:3816-3824.
[70] Baraldo et al. (2004) Infect Immun 72(8):4884-7.
[71] EP-A-0594610.
[72] Ruan et al. (1990) J Immunol 145:3379-3384.
[73] WO00/56360.
[74] Kuo et al. (1995) Infect Immun 63:2706-13.
[75] Michon et al. (1998) Vaccine. 16:1732-41.
[76] WO02/091998.
[77] WO01/72337
[78] WO00/61761.
[79] WO00/33882
[80] WO99/42130
[81] WO2007/000341.
[82] Bardotti et al. (2008) Vaccine 26:2284-96.
[83] Mol. Immunol., 1985, 22, 907-919
[84] EP-A-0208375
[85] Bethell G.S. et al., J. Biol. Chem., 1979, 254, 2572-4
[86] Hearn M.T.W., J. Chromatogr., 1981, 218, 509-18
[87] WO00/10599
[88] Gever et al., Med. Microbiol. Immunol, 165 : 171-288 (1979).
[89] US patent 4,057,685.
[90] US patents 4,673,574; 4,761,283; 4,808,700.
[91] US patent 4,459,286.
[92] US patent 5,204,098
[93] US patent 4,965,338
[94] US patent 4,663,160.
[95] WO2007/000343.
[96] WO2007/000342.
[97] WO2007/000322.
[98] WO90/14837.
[99] Podda & Del Giudice (2003) Expert Rev Vaccines 2:197-203.
[100] Podda (2001) Vaccine 19: 2673-2680.
[101] Vaccine Design: The Subunit and Adjuvant Approach (eds. Powell & Newman) Plenum Press 1995 (ISBN 0-306-44867-X).
[102] Vaccine Adjuvants: Preparation Methods and Research Protocols (Volume 42 of Methods in Molecular Medicine series). ISBN: 1-59259-083-7. Ed. O'Hagan.
[103] Allison & Byars (1992) Res Immunol 143:519-25.
[104] Hariharan et al. (1995) Cancer Res 55:3486-9.
[105] US-2007/014805.
[106] WO95/11700.
[107] US patent 6,080,725.
[108] WO2006/113373.
[109] WO2005/097181.
[110]GB-A-2220211.
[111] US-5472706
[112] WO2006/110603.
[113] Gennaro (2000) Remington: The Science and Practice of Pharmacy. 20th edition, ISBN: 0683306472.
[114] RIVM report 124001 004.
[115] RIVM report 000012 003.
[116] Bakke et al. (2001) Infect. Immun. 69:5010-5015.
[117] WO01/30390.
[118] *http:*//*neisseria.org*/*nm*/*typing*/*mlst*/
[119] Tettelin et al. (2000) Science 287:1809-1815.
[120] Pettersson et al. (1994) Microb Pathog 17(6):395-408.
[121] Welsch et al. (2002) Thirteenth International Pathogenic Neisseria Conference, Norwegian Institute of Public Health, Oslo, Norway; Sept. 1-6, 2002. Genome-derived antigen (GNA) 2132 elicits protective serum antibodies to groups B and C Neisseria meningitidis strains*.*
[122] Santos et al. (2002) Thirteenth International Pathogenic Neisseria Conference, Norwegian Institute of Public Health, Oslo, Norway; Sept. 1-6, 2002. Serum bactericidal responses in rhesus macaques immunized with novel vaccines containing recombinant proteins derived from the genome of N. meningitidis*.*
[123] WO2007/000327.
[124] WO2007/071707

## Claims

1. A lyophilised antigenic composition comprising (i) an immunogen for raising an immune response against *Neisseria meningitidis* serogroup B, and (ii) a conjugated capsular saccharide from one or more of *Neisseria meningitidis* serogroups A, C, W135 and/or Y.

2. A kit comprising: (i) a first container containing an adjuvant comprising an oil-in-water emulsion; and (ii) a second container containing a lyophilised antigenic composition comprising an immunogen for raising an immune response against *Neisseria meningitidis* serogroup B.

3. The kit of claim 2, wherein the lyophilised antigenic composition in the second container further comprises a conjugated capsular saccharide from one or more of *N.meningitidis* serogroups A, C, W135 and/or Y.

4. A method for preparing an immunogenic composition, comprising a step of mixing: (i) an adjuvant comprising an oil-in-water emulsion; and (ii) a lyophilised antigenic composition comprising an immunogen for raising an immune response against *Neisseria meningitidis* serogroup B.

5. The method of claim 4, wherein the lyophilised antigenic composition further comprises a conjugated capsular saccharide from one or more of *N.meningitidis* serogroups A, C, W135 and/or Y.

6. The method of claim 4, wherein the lyophilised antigenic composition is according to claim 3.

7. The composition, kit, or method of any preceding claim, wherein the lyophilised antigenic composition comprises membrane vesicles from a serogroup B strain *of N.meningitidis.*

8. The composition, kit, or method of any preceding claim, wherein the lyophilised antigenic composition comprises recombinant proteins of a serogroup B strain of *N.meningitidis.*

9. The composition, kit, or method of any preceding claim, wherein the lyophilised antigenic composition comprises a lipooligosaccharide from a serogroup B strain of *N.meningitidis.*
